(19) **Europäisches Patentamt / European Patent Office / Office européen des brevets**

(11) **EP 4 200 594 B1**

(12) **FASCICULE DE BREVET EUROPEEN**

(45) Date de publication et mention
de la délivrance du brevet:
**03.06.2026 Bulletin 2026/23**

(21) Numéro de dépôt: **21765921.8**

(22) Date de dépôt: **20.08.2021**

(51) Classification Internationale des Brevets (IPC):
**G01N 21/3563** *(2014.01)* **G01N 21/27** *(2006.01)*
**G01N 21/59** *(2006.01)* **G01N 21/88** *(2006.01)*
**G01N 21/64** *(2006.01)* **G01N 33/02** *(2006.01)*
**G01N 33/10** *(2006.01)* **G01N 21/03** *(2006.01)*

(52) Classification Coopérative des Brevets (CPC):
**G01N 21/3563; G01N 21/645; G01N 33/10;**
G01N 2021/0339; G01N 2021/036;
G01N 2021/0367; G01N 2021/8466

(86) Numéro de dépôt international:
**PCT/EP2021/073197**

(87) Numéro de publication internationale:
**WO 2022/038287 (24.02.2022 Gazette 2022/08)**

(54) **DISPOSITIF D'ANALYSE SPECTROSCOPIQUE D'UN ECHANTILLON ET PROCEDE D'ANALYSE D'UN ECHANTILLON AU MOYEN D'UN TEL DISPOSITIF**

VORRICHTUNG ZUR SPEKTROSKOPISCHEN ANALYSE EINER PROBE UND VERFAHREN ZUR ANALYSE EINER PROBE MIT EINER SOLCHEN VORRICHTUNG

DEVICE FOR SPECTROSCOPIC ANALYSIS OF A SAMPLE AND METHOD FOR ANALYSING A SAMPLE BY MEANS OF SUCH A DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.08.2020 FR 2008613**

(43) Date de publication de la demande:
**28.06.2023 Bulletin 2023/26**

(73) Titulaire: **Spectralys Innovation**
**92600 Asnières-sur-Seine (FR)**

(72) Inventeurs:
- **ACHARID, Abdelhaq**
**95270 LUZARCHES (FR)**
- **BIRLOUEZ, Inès**
**95120 ERMONT (FR)**
- **BOUTAOUAKOU, Papus**
**77700 CHESSY (FR)**
- **CHARLES-FRANCOIS, Olivier**
**75011 PARIS (FR)**
- **LEGRAND, Elliott**
**93170 BAGNOLET (FR)**
- **MESSAOUDI, Monji**
**93300 AUBERVILLIERS (FR)**
- **ODDOS, Stéphane**
**92100 BOULOGNE BILLANCOURT (FR)**
- **VOISIN, Clément**
**62690 HERMAVILLE (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
WO-A1-2020/012029    FR-A1- 3 047 313
US-A- 5 208 649    US-B2- 8 946 618

EP 4 200 594 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**Domaine technique de l'invention**

L'invention se rapporte au domaine technique de l'analyse spectroscopique, et en particulier de l'analyse par spectroscopie infrarouge et par spectroscopie de fluorescence. Le couplage de ces deux technologies permet en effet d'apporter des informations complémentaires, voire synergiques, sur le même échantillon.

L'invention concerne un dispositif d'analyse d'un échantillon. L'invention concerne en outre un procédé d'analyse d'un échantillon au moyen d'un tel dispositif.

Dans le cadre de l'invention, l'échantillon est plus particulièrement un échantillon hétérogène qui peut être solide (grains, broyat de biscuit ou de chips, pâte) ou pulvérulent (farine, poudre de lait). Un échantillon hétérogène au sens de l'invention est un échantillon comprenant des éléments de tailles variées et très absorbants. Par exemple, l'échantillon peut contenir des impuretés. Cela étant dit, dans le cas d'un échantillon de grains, l'hétérogénéité de l'échantillon peut tenir aussi à sa granulométrie.

L'invention trouve son application dans l'industrie agro-alimentaire et en particulier dans l'industrie céréalière ou laitière. L'invention vise en effet à permettre l'analyse d'un échantillon à différents stades de son élaboration par les professionnels concernés. Dans l'industrie agroalimentaire, en particulier céréalière ou laitière, les procédures industrielles nécessitent une connaissance précise des propriétés et des indices de la qualité des échantillons analysés (indice de chute de Hagberg du blé, alvéographe, farinographe, ou encore tests de panification sur pâte). Dans ce cadre, l'analyse d'échantillons au moyen de techniques spectroscopiques permet d'extraire en quelques dizaines de secondes, tout un ensemble d'informations physico-chimiques qui se traduisent aisément en diverses fonctionnalités du produit, au travers de la construction de calibrations entre les informations spectrales et les critères décrivant les fonctionnalités.

**Arrière-plan technique**

On connait des dispositifs de spectroscopie ayant recours à différentes méthodes d'analyse permettant d'obtenir des informations physico-chimiques sur des échantillons. Les méthodes d'analyse dont il s'agit sont classiquement la spectroscopie de fluorescence et la spectroscopie infrarouge.

Le document WO 2019/118800 A1 divulgue un appareil de mesure disponible commercialement qui permet d'effectuer des analyses physico-chimiques sur des échantillons au moyen de mesures par spectroscopie infrarouge et par spectroscopie de fluorescence. L'appareil comprend une source unique et un monochromateur permettant de modifier la longueur d'onde du faisceau émis sur une gamme spectrale appropriée pour effectuer les deux types de mesure. L'appareil comprend un jeu de miroirs et de réflecteurs permettant au faisceau émis par la source d'emprunter divers chemins optiques dont un au moins est dédié aux mesures par spectroscopie infrarouge et un autre au moins aux mesures par spectroscopie de fluorescence.

Si cet appareil permet effectivement d'effectuer les mesures sur le même échantillon, le volume d'échantillon qui peut être analysé est de 1 cm$^3$. En effet, ce type d'appareil est essentiellement utilisé dans les milieux académiques où les échantillons observés sont des échantillons modèles homogènes, souvent de très petite taille. Cependant, si ce volume était exprimé en nombre de grains cela ne représenterait que quelques grains, et de plus de très petite taille. Or, dans l'industrie agroalimentaire, en particulier céréalière ou laitière, les échantillons étudiés - grains, pâtes, poudres, etc. - sont non seulement généralement très hétérogènes en termes de taille, de forme, voire même de composition mais en plus ils ont un volume beaucoup plus important, ceci afin qu'ils soient représentatifs du contenu d'un silo (plusieurs milliers de mètres cubes). En outre, si cet appareil permet d'analyser des échantillons très peu absorbants tels que des échantillons liquides, des couches minces, des couches de papier, des pierres précieuses, etc., il n'est pas adapté pour analyser des échantillons très absorbants.

Pour mesurer les paramètres d'échantillons hétérogènes, tels que définis ci-dessus, avec précision et en conformité avec les normes industrielles, des dispositifs adaptés ont été développés.

On connait du document EP 1 850 117 A1 un dispositif d'analyse spectroscopique permettant d'effectuer des mesures sur de tels échantillons à partir des deux types de mesures. Le dispositif comprend un premier module dédié à l'analyse par spectroscopie infrarouge. Ce module comprend une chambre munie d'une source émettant un rayonnement électromagnétique dans le proche infrarouge et l'infrarouge pour éclairer l'échantillon, d'un détecteur du type monochromateur à réseau ou un filtre servant à la lecture du spectre de transmittance de l'échantillon et d'un emplacement destiné à accueillir ledit échantillon. Le dispositif comprend en outre un deuxième module spécifiquement dédié à l'analyse par spectroscopie de fluorescence. Ce module comprend une chambre comportant une source émettant un rayonnement électromagnétique destiné à éclairer l'échantillon de sorte que ce dernier, sous l'effet de ce rayonnement, émette un signal typique de fluorescence. Le module comprend également un détecteur de fluorescence apte à mesurer le signal émis par l'échantillon et un emplacement réservé à l'échantillon.

Deux modes opératoires associés chacun à une configuration sont alors possibles. Dans une première configuration, un échantillon de matière est préalablement séparé en deux échantillons chacun étant déplacé vers un module spécifique dans le but d'effectuer un type de mesure en particulier, tandis que dans une deuxième configuration ledit échantillon de matière est successivement déplacé d'un module à l'autre.

Lorsque l'échantillon est séparé en deux échantillons, des manipulations supplémentaires sont nécessaires pour séparer l'échantillon. De plus, le volume d'échantillon analysé par spectroscopie infrarouge n'est jamais le même que le volume d'échantillon analysé par spectroscopie de fluorescence puisque les deux échantillons sont toujours distincts, quand bien même ils proviennent du même échantillon de départ, du fait même de l'hétérogénéité de cet échantillon de départ.

Lorsque l'échantillon est déplacé successivement entre les deux modules de l'appareil, des manipulations supplémentaires sont requises pour déplacer l'échantillon d'un module à un autre. De plus, toujours selon cette configuration, il n'est pas possible de garantir qu'un volume de l'échantillon analysé par spectroscopie infrarouge est le même volume d'une prise d'essai indépendante qui est analysé par spectroscopie de fluorescence recouvrent la même réalité physico-chimique, du fait de la dispersion. En effet, si l'échantillon solide est de nature hétérogène alors il y a toujours un doute que le sous échantillon analysé ne soit pas représentatif de l'échantillon initial. Et même si l'échantillon est représentatif de l'échantillon initial, le positionnement de l'échantillon dans la chambre de mesure n'est pas le même lors du déplacement de l'échantillon entre les deux modules. Cela aura nécessairement un impact sur la mesure.

Les dispositifs d'analyse spectroscopique précités souffrent donc de plusieurs inconvénients puisque soit ils ne permettent pas d'analyser des échantillons hétérogènes, de tailles variées et très absorbants, soit ils ne permettent pas de réaliser des mesures par spectroscopie infrarouge et par spectroscopie de fluorescence sur le même échantillon, sans avoir à déplacer l'échantillon ou à séparer l'échantillon en deux.

Les documents FR 3 047 313 A1 et WO 2020/012029 A1 divulguent des dispositifs d'analyse d'un échantillon hétérogène.

La conséquence de cela est que les dispositifs de l'art antérieur ne permettent pas d'effectuer un couplage optimisé des données obtenues par la spectroscopie infrarouge et par la spectroscopie de fluorescence puisque l'image de l'échantillon mesurée par spectroscopie infrarouge ne correspond à l'image de cet échantillon mesurée par spectroscopie de fluorescence.

**Résumé de l'invention**

L'invention permet de surmonter les inconvénients précités et propose à cet effet un dispositif d'analyse d'un échantillon hétérogène, ledit dispositif étant caractérisé en ce qu'il comprend :

- un module de mesure comprenant :

  ○ un réservoir configuré pour accueillir ledit échantillon et muni d'une première paroi et d'une deuxième paroi opposée à la première paroi,
  ○ un premier sous-ensemble de spectroscopie infrarouge comprenant une première source d'excitation configurée pour émettre un rayonnement électromagnétique dans le domaine de l'infrarouge et/ou du proche infrarouge vers la première paroi du réservoir, ladite première paroi étant transparente pour le rayonnement électromagnétique infrarouge, et un premier moyen d'acquisition de spectres de transmittance,
  ○ un deuxième sous-ensemble de spectroscopie de fluorescence comprenant au moins une deuxième source d'excitation configurée pour émettre un rayonnement électromagnétique dans le domaine ultraviolet et/ou visible vers la deuxième paroi du réservoir, ladite deuxième paroi étant transparente pour les rayonnements électromagnétiques, de sorte que le volume de l'échantillon susceptible d'être éclairé par la première source d'excitation corresponde au moins en partie au volume de l'échantillon susceptible d'être éclairé par la deuxième source d'excitation, et un deuxième moyen d'acquisition de spectres de fluorescence dudit échantillon,
  ○ le premier sous-ensemble comprenant un élément optique diffusant et transparent pour le rayonnement électromagnétique émis par ladite première source d'excitation, ledit élément optique étant positionné entre la première source d'excitation et la première paroi du réservoir ou entre la deuxième paroi du réservoir et le premier moyen d'acquisition, hors d'un trajet optique du rayonnement électromagnétique émis par la deuxième source d'excitation et
  ○ hors d'un angle solide de collecte d'un signal de fluorescence émis par l'échantillon lorsqu'il est exposé au rayonnement électromagnétique émis par la deuxième source d'excitation, et

- un module de traitement connecté au module de mesure par un réseau de communication et comprenant un processeur configuré pour analyser les données obtenues par spectroscopie infrarouge et spectroscopie de fluorescence,
- un système de retenue et une ouverture du module de mesure servant à y distribuer l'échantillon ou un logement recevant un système de retenue de l'échantillon, ledit logement comprenant des faces intérieures sur lesquelles est plaqué le système de retenue, le système de retenue comprenant :

- le réservoir, ledit réservoir comprenant une première partie vitrée munie de la première paroi et une deuxième partie vitrée munie de la deuxième paroi, ladite première partie vitrée étant montée de manière amovible sur ladite deuxième partie vitrée,
- un bloc support configuré pour positionner le système de retenue dans le logement du système de mesure ou au niveau de l'ouverture du module de mesure servant à y distribuer l'échantillon, le bloc support comprenant une portion de raccordement et une base de préhension du système de retenue formant un coude avec la portion de raccordement dans le dispositif,
- une portion amovible comprenant un logement recevant le réservoir, ladite deuxième partie vitrée étant fixée à la portion amovible, ladite portion amovible étant reliée de manière amovible par rapport à la portion de raccordement, la base de préhension venant en butée dans le logement du module de mesure ou se positionnant au niveau de l'ouverture du module de mesure et permettant à la portion de raccordement et à la portion amovible de s'étendre dans le module de mesure sur le chemin optique, lorsque le système de retenue est inséré dans le module de mesure,
- une portion articulée comprenant une ouverture, ladite portion articulée étant montée pivotante sur la portion support et apte à passer d'une position de montage dans laquelle elle est éloignée de la portion amovible à une position d'utilisation dans laquelle elle est rabattue sur la portion amovible, ladite ouverture étant en vis-à-vis du réservoir, ladite portion articulée comprenant des moyens compressibles permettant le plaquage du système de retenue contre les faces intérieures dudit logement du module de mesure recevant le système de retenue lorsque ladite portion articulée est en position d'utilisation.

On dispose ainsi d'un dispositif permettant d'analyser les échantillons hétérogènes solides ou pulvérulents, de tailles variées et très absorbants tels que les graines, les farines, les pâtes, etc. De plus, le dispositif permet de mettre en œuvre des mesures de spectroscopie infrarouge et de spectroscopie de fluorescence dans un unique module sans avoir à déplacer l'échantillon à analyser d'un module à un autre ou à le séparer en deux pour effectuer les deux types de mesures. Dans une telle configuration, le volume de l'échantillon analysé par spectroscopie infrarouge correspond toujours au moins en partie au volume de l'échantillon analysé par spectroscopie de fluorescence, ce qui permet de corréler les données issues des deux types de mesures. De plus, le nombre de manipulations est réduit ce qui permet de réduire l'impact sur le temps des mesures et le temps passé à réaliser les mesures. Cela fait du dispositif selon l'invention un dispositif particulièrement adapté à l'analyse physico-chimique en milieu industriel.

La problématique liée à la mesure de transmittance d'échantillons très absorbants a été résolue en ayant recours à l'élément optique diffusant et transparent.

De manière classique, lorsqu'on souhaite effectuer des mesures par spectroscopie infrarouge (transmittance et/ou réflectance) sur des échantillons hétérogènes, de tailles variées et très absorbants, il existe une telle différence d'intensité lumineuse transmise entre la mesure sans échantillon et la mesure avec l'échantillon que le rapport d'intensité entre la mesure sans échantillon et la mesure avec échantillon est de l'ordre de 20 000. Pour corriger ce problème, il existe deux solutions naturelles. La première consiste à ajouter un élément absorbant sur le trajet optique entre la source et le moyen de détection lors de la mesure sans échantillon. L'élément absorbant permet d'obtenir des intensités comparables avec et sans l'échantillon mais altère le chemin optique entre les deux mesures. Une « composante » supplémentaire doit donc être prise en compte dans les spectres obtenus et éventuellement supprimée, ce qui peut s'avérer complexe. La seconde solution consiste à altérer, i.e. atténuer, le spectre de la source elle-même en faisant varier l'intensité lumineuse entre les deux mesures. Le problème qui se pose alors est celui de la répétabilité des mesures.

L'élément optique diffusant et transparent permet de rendre diffus le rayonnement émis par la source lors de la mesure par spectroscopie infrarouge sans échantillon et permet donc de réduire l'intensité de la lumière transmise au détecteur lors de cette mesure. En revanche, lors de la mesure avec l'échantillon, comme il rend diffus le rayonnement émis par la source mais dans une moindre mesure que l'échantillon hétérogène lui-même, du fait de la présence d'éléments de tailles variées et très absorbants, il constitue un élément neutre et passif puisqu'il atténue très peu le rayonnement de la source en comparaison de l'atténuation due à l'échantillon lui-même, sans perte d'information. Ainsi, il est possible de significativement réduire le rapport d'intensité entre les deux mesures sans rallonger le temps de mesure, sans rallonger la longueur du chemin optique, sans ajouter d'élément à la source d'excitation et/ou altérer le spectre optique de la source d'excitation. Ainsi, par rapport à certaines solutions de l'art antérieur, l'invention permet d'améliorer la répétabilité des mesures, fournit plus de robustesse et permet d'éviter d'avoir à modifier la source entre deux mesures.

Cependant, l'intégration d'un tel élément optique diffusant dans la chambre unique de mesure du dispositif alors même que doivent y être effectuées également des mesures de fluorescence sur le même échantillon, qui sont par nature très sensibles à la diffusion, est loin d'être évident. Pour répondre à cette problématique, l'élément optique diffusant est positionné entre la première source d'excitation et la première paroi du réservoir ou entre la deuxième paroi du réservoir et le premier moyen d'acquisition, hors d'un trajet optique du rayonnement électromagnétique émis par la deuxième source d'excitation et hors d'un angle solide de collecte d'un signal de fluorescence émis par l'échantillon lorsqu'il est exposé rayonnement électromagnétique émis par la deuxième source d'excitation. En étant ainsi positionnée, il est possible d'effectuer les mesures par spectroscopie infrarouge sur des échantillons hétérogènes, de tailles variées et très absorbants, sans entraver les mesures par spectroscopie de fluorescence. Le dispositif ainsi revendiqué permet donc de réaliser des mesures par spectroscopie infrarouge et spectroscopie de fluorescence fiables et avec une précision élevée sur le même échantillon.

Le dispositif de l'invention permet d'améliorer la conformité entre l'image de l'échantillon mesurée par spectroscopie infrarouge et l'image de l'échantillon mesurée par spectroscopie de fluorescence et permet ainsi d'optimiser le couplage des données obtenues par spectroscopie infrarouge et par spectroscopie de fluorescence.

Selon différentes caractéristiques de l'invention qui pourront être prises ensemble ou séparément :

- l'élément optique constitue la première paroi dudit réservoir ;

- l'élément optique est positionné entre la première source d'excitation et la première paroi du réservoir, sur le chemin optique entre la première source d'excitation et le premier moyen d'acquisition,

- la première paroi du réservoir est mobile le long d'un axe orthogonal à un plan passant par la première paroi,

- la deuxième paroi du réservoir est anti-réfléchissante pour le rayonnement électromagnétique émis par la deuxième source,

- ladite deuxième source est située entre la deuxième paroi et le premier moyen d'acquisition ;

- un axe passant par le deuxième moyen d'acquisition et un plan médian et sensiblement orthogonal à la deuxième paroi du réservoir forme un angle $\alpha$ par rapport à un axe passant par la première source d'excitation et le deuxième moyen d'acquisition .

- la première source d'excitation émet un rayonnement électromagnétique polychromatique large-bande,

- ladite première source consiste en une source à incandescence halogène haute puissance.

- la(es) deuxième(s) source(s) d'excitation émet(tent) un rayonnement électromagnétique monochromatique,

- ladite(lesdites) deuxième(s) source(s) consiste en une(des) diodes électroluminescentes,

L'invention concerne en outre un procédé d'analyse d'un échantillon au moyen d'un dispositif tel que décrit précédemment, ledit procédé comprenant les étapes suivantes :

A) acquérir un spectre de transmittance dudit échantillon avec le premier sous-ensemble de spectroscopie infrarouge,

B) acquérir des spectres de fluorescence dudit échantillon avec le deuxième sous-ensemble de spectroscopie de fluorescence,

C) analyser les données obtenues par spectroscopie infrarouge et spectroscopie de fluorescence au moyen du module de traitement, un processeur étant configuré pour déterminer au moins un critère caractérisant ledit échantillon à partir des données issues de l'analyse,

D) coupler les données obtenues par spectroscopie infrarouge et spectroscopie de fluorescence, au niveau spectral, au moyen du module de traitement, par concaténation des spectres, traités préalablement, et par association de scores issus de la décomposition de chaque spectre, pour construire des régressions linéaires ou des modèles non linéaires et obtenir des calibrations d'un critère descriptif de l'état de l'échantillon, comme un critère de qualité technologique, sensoriel ou nutritionnel et sanitaire.

**Brève description des figures**

D'autres objets et caractéristiques de l'invention apparaîtront plus clairement dans la description qui suit, faite en référence aux figures annexées, dans lesquelles :

[Fig. 1a] La figure 1a est une représentation schématique d'un dispositif d'analyse selon un premier mode de réalisation de l'invention dans lequel l'élément optique est positionné entre la première source d'excitation et la première paroi du réservoir, l'élément optique faisant partie du réservoir,

[Fig. 1b] La figure 1b est une représentation schématique d'un dispositif d'analyse selon un mode de réalisation de l'invention dans lequel l'élément optique est positionné entre la première source d'excitation et la première paroi du réservoir, l'élément optique étant un élément distinct du réservoir,

[Fig. 1c] La figure 1c est une représentation schématique d'un dispositif d'analyse selon un mode de réalisation de l'invention dans lequel l'élément optique est situé entre une deuxième paroi du réservoir et une première source,

[Fig. 2a] La figure 2a est une illustration, en vue latérale, du dispositif d'analyse de la figure 1a,

[Fig. 2b] La figure 2b est une illustration, en vue latérale, du dispositif d'analyse de la figure 1b,

[Fig. 3] La figure 3 est une illustration, en perspective, d'un support pour la deuxième source d'excitation,

[Fig. 4a] La figure 4a est une illustration, en perspective, d'un système de retenue d'un échantillon pulvérulent, visqueux ou pâteux pour le dispositif d'analyse d'un échantillon selon l'invention,

[Fig. 4b] La figure 4b est une illustration, en perspective, du système de retenue de la figure 4, montrant une des faces du système de retenue,

[Fig. 5] La figure 5 illustre une vue approchée et éclatée du réservoir du système de retenu illustré sur les figures 4a et 4b,

[Fig. 6a] La figure 6a illustre des composantes liées à la fluorescence pour des spectres bruts obtenus lors de l'étape B) par application du procédé selon l'invention sur un échantillon d'orge (en ligne solide) et par spectroscopie de fluorescence par la méthode de référence connue de l'art antérieur (en ligne pointillée) pour une longueur d'onde d'excitation de 340 nm,

[Fig. 6b] La figure 6b correspond aux spectres de la figure 6a après filtrage gaussien,

[Fig. 6c] La figure 6c illustre une série de spectres obtenus lors de l'étape B) par répétition du procédé selon l'invention sur un même échantillon d'orge pour une longueur d'onde d'excitation de 340 nm,

[Fig. 6d] La figure 6d illustre une série de spectres obtenus par spectroscopie de fluorescence par la méthode de référence connue de l'art antérieur sur un même échantillon d'orge pour une longueur d'onde d'excitation de 340 nm,

[Fig. 6e] La figure 6e illustre les résidus de chacun des spectres de la figure 6c par rapport au spectre moyen,

[Fig. 6f] La figure 6f illustre les résidus de chacun des spectres de la figure 6d par rapport au spectre moyen,

[Fig. 7a] La figure 7a illustre des composantes liées à la fluorescence pour des spectres bruts obtenus lors de l'étape B) par application du procédé selon l'invention sur un échantillon d'orge (en ligne solide) et par spectroscopie de fluorescence par la méthode de référence connue de l'art antérieur (en ligne pointillée) pour une longueur d'onde d'excitation de 385 nm,

[Fig. 7b] La figure 7b correspond aux spectres de la figure 7a après filtrage gaussien,

[Fig. 7c] La figure 7c illustre une série de spectres obtenus lors de l'étape B) par répétition du procédé selon l'invention sur un même échantillon d'orge pour une longueur d'onde d'excitation de 385 nm,

[Fig. 7d] La figure 7d illustre une série de spectres obtenus par spectroscopie de fluorescence par la méthode de référence connue de l'art antérieur sur un même échantillon d'orge pour une longueur d'onde d'excitation de 385 nm,

[Fig. 7e] La figure 7e illustre les résidus de chacun des spectres de la figure 7c par rapport au spectre moyen,

[Fig. 7f] La figure 7f illustre les résidus de chacun des spectres de la figure 7d par rapport au spectre moyen,

[Fig. 8a] La figure 8a illustre une régression PLS (RMSECV = 0,022%) obtenue en reliant les valeurs mesurées par spectroscopie infrarouge par la méthode de référence connue de l'art antérieur (axe des abscisses) et les valeurs prédites par validation croisée (axe des ordonnées) pour chaque échantillon d'un lot de 204 farines,

[Fig. 8b] La figure 8b illustre une régression MLR (RMSECV = 0,018%) obtenue en reliant les valeurs mesurées par spectroscopie de fluorescence par la méthode de référence connue de l'art antérieur (axe des abscisses) et les valeurs prédites par validation croisée (axe des ordonnées) pour chaque échantillon d'un lot de 204 farines,

[Fig. 9] La figure 9 illustre une régression PLS successive (RMSECV = 0,008%) réalisée en utilisant conjointement les valeurs mesurées par spectroscopie infrarouge et spectroscopie de fluorescence (axe des abscisses) selon le procédé de l'invention de cendres sur de la farine de blé et les valeurs prédites par validation croisée (axe des ordonnées) pour chaque échantillon d'un lot de 204 farines afin de construire une calibration.

## Description détaillée de l'invention

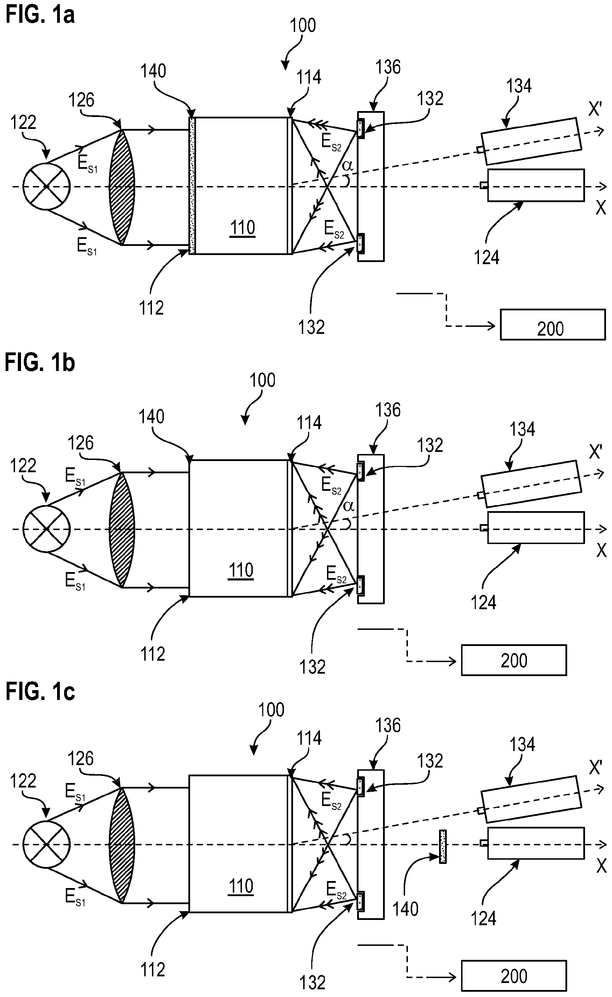

En référence à la figure 1a, l'invention concerne un dispositif 1 d'analyse d'un échantillon S comprenant un module 100 de mesure et un module 200 de traitement connecté au module 100 de mesure.

Le module 100 de mesure comprend un réservoir 110 pour accueillir l'échantillon S, un premier sous-ensemble 120 de spectroscopie infrarouge et un deuxième sous-ensemble 130 de spectroscopie de fluorescence.

Bien que le premier sous-ensemble 120 de spectroscopie infrarouge et le deuxième sous-ensemble 130 de spectroscopie de fluorescence diffèrent par les éléments qui les composent, leurs éléments sont en revanche localisés dans un module commun. Ainsi, contrairement aux systèmes connus, le premier sous-ensemble 120 et le deuxième sous-ensemble 130 ne forment pas des sous-modules délimités spatialement l'un de l'autre telles deux boîtes simplement juxtaposées mais bien des sous-ensembles dont les éléments sont agencés de manière optimale dans l'unique module 100 de mesure.

Comme nous le verrons en détail dans ce qui suit, cet agencement optimal permet d'effectuer des mesures par spectroscopie infrarouge et spectroscopie de fluorescence au moyen du premier sous-ensemble 120 et du deuxième sous-ensemble 130 respectivement sans avoir à déplacer ou séparer l'échantillon, ce qui permet de réduire le nombre de manipulations, de mener une analyse sur rigoureusement le même échantillon et également d'améliorer la répétabilité des mesures. En outre, le délai pour effectuer les deux mesures sur le même échantillon s'établit à, à peine plus d'une minute, ce qui permet à l'industriel de pouvoir en déduire les critères quantitatifs des grains dans un temps court qui peut être qualifié de temps réel. Comme cela sera mieux décrit dans la suite, l'agencement du réservoir 110 et des éléments des premier et deuxième sous-ensembles 120, 130 au sein du module 100 de mesure selon l'invention est particulièrement ingénieux puisqu'il permet d'effectuer des mesures par spectroscopie infrarouge et spectroscopie de fluorescence en temps réduit au moyen de l'unique module 100 de mesure et sur un même échantillon S.

Le réservoir 110 pour accueillir l'échantillon est muni d'une première paroi 112 et d'une deuxième paroi 114 opposée à la première paroi 112.

Le réservoir 110 peut être de toute forme dès lors qu'il comprend une première et une deuxième parois 112, 114 telles que définies précédemment. Par exemple, le réservoir 110 peut être de forme parallélépipédique. Dans ce cas, les première et deuxième parois 112, 114 peuvent alors être formées au niveau de deux faces opposées du parallélépipède. Le réservoir 110 peut également être de forme cylindrique. Dans ce cas, les première et deuxième parois 112, 114 correspondent alors aux bases du cylindre. Il s'agit là d'exemples non limitatifs.

Le réservoir 110 délimite avantageusement un volume destiné à accueillir un échantillon S. Le volume du réservoir 110 est avantageusement de l'ordre de 100 mL. En outre, chacune desdites première et deuxième parois 112, 114 confère une surface d'éclairement de quelques dizaines de $cm^2$, de préférence d'environ 20 $cm^2$, de grains de céréales. Ce qui importe est que la surface éclairée permette d'effectuer une mesure représentative de l'échantillon en dépit de son hétérogénéité tout en permettant d'avoir un temps de mesure approprié (environ 1 minute) et une taille de dispositif appropriée (encombrement limité). Ainsi, on évite d'utiliser plusieurs sous-échantillons de plus petites tailles, qui non seulement sont susceptibles de ne pas être suffisamment représentatifs du lot analysé mais également qui requièrent un temps de mesure et d'analyse plus important, etc. Les dimensions du réservoir sont ainsi suffisantes pour sonder une quantité de matière représentative de l'ensemble du produit dont l'industriel veut connaître les propriétés à partir de mesures de spectroscopie infrarouge et de spectroscopie de fluorescence. Les dimensions du réservoir 110 sont également appropriées pour effectuer à la fois les mesures par les deux types de spectroscopies sans pour autant augmenter indûment la longueur du chemin optique entre les sources et les détecteurs des premier et deuxième sous-ensembles 120, 130. Au-delà de ces considérations, le réservoir 110 peut avoir toute dimension que l'utilisateur du dispositif jugera adaptée aux échantillons qu'il souhaite analyser.

De manière préférentielle, le réservoir 110 peut également être muni d'une paroi mobile permettant d'adapter le volume dudit réservoir selon le type d'échantillon hétérogène (granuleux, pulvérulent ou pâteux). La paroi mobile peut être n'importe laquelle des parois du réservoir 110. Ainsi, il est possible de réduire/augmenter la profondeur du réservoir, qui détermine le trajet optique du faisceau lumineux, selon que l'on analyse des grains de petite/grande taille qui présentent des niveaux d'absorbance de la lumière inversement proportionnels à leur taille. S'il s'agit de la première paroi 112 du réservoir, ladite première paroi 112 est alors, de préférence, mobile le long d'un axe X orthogonal à un plan passant par ladite première paroi 112. Selon l'exemple de réalisation illustré sur la figure 1a, la première paroi 112 s'étend le long d'un plan sensiblement vertical. Ainsi, l'axe le long duquel se déplace la paroi mobile 112 est un axe horizontal.

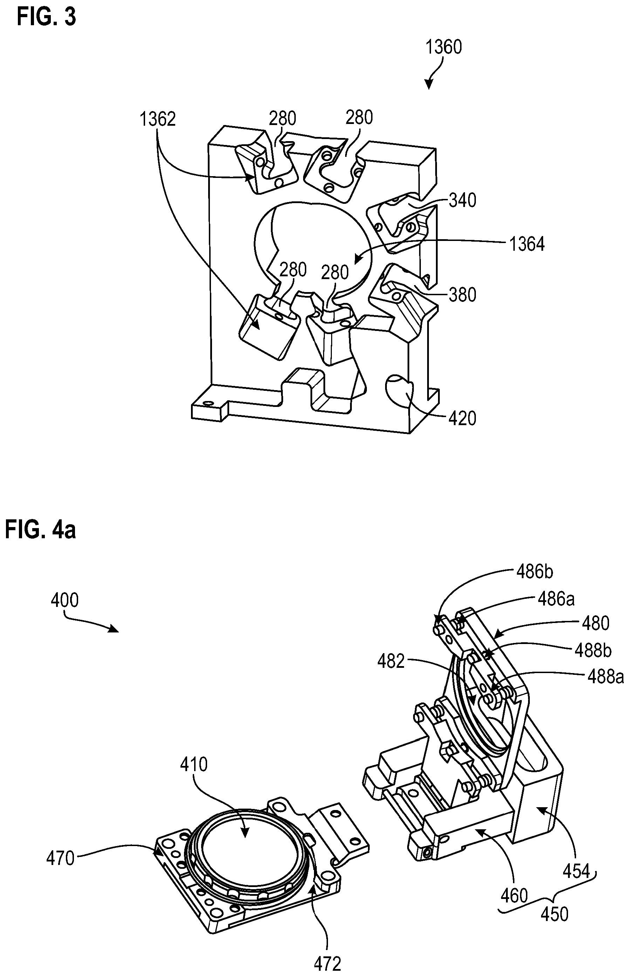

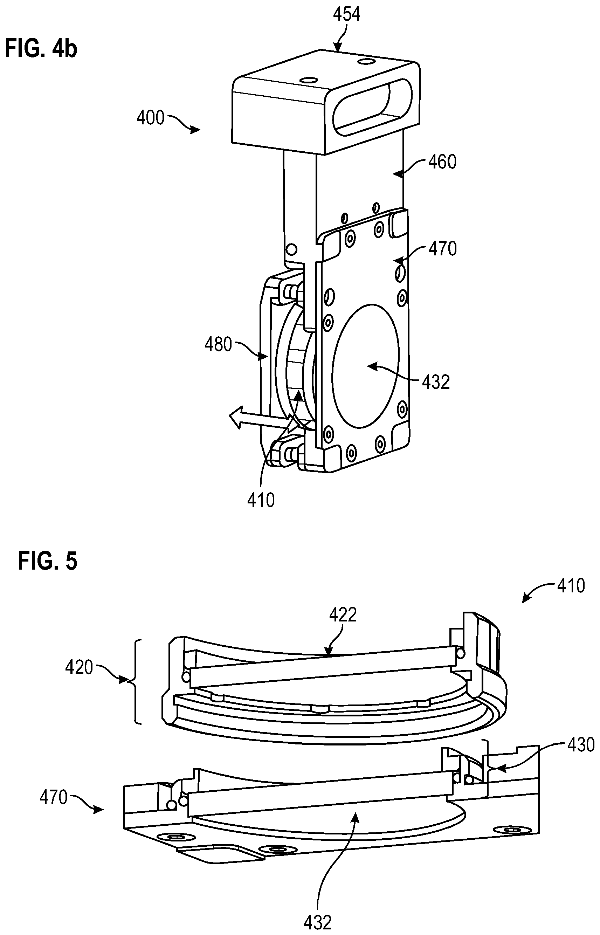

Selon l'invention comme illustrée aux figures 4a et 4b et 5, le dispositif 1 d'analyse comprend un système de retenue 400 de l'échantillon configuré pour accueillir et doser aussi bien des échantillons granuleux, pulvérulents que des échantillons pâteux.

Le système de retenue 400 comprend un réservoir 410 pour accueillir l'échantillon, mieux visible sur la figure 5. Le réservoir 410 comprend une première partie vitrée 420 munie d'une première paroi 422. Il comprend en outre une deuxième partie vitrée 430 munie d'une deuxième paroi 432. Précisons que le terme « vitrée » n'est nullement limitatif dans le cadre de la présente invention et qu'il n'implique aucunement que le matériau dont sont faites les parois transparentes est nécessairement du verre. Il peut en effet s'agir d'un tout autre matériau dès lors qu'il présente les mêmes propriétés que les parois 122, 132 décrites précédemment. La deuxième paroi 432 est séparée de la première partie vitrée 420 un volume vide destiné à accueillir l'échantillon. La première partie vitrée 420 est montée de manière amovible sur ladite deuxième partie vitrée 430, c'est-à-dire que la première partie vitrée 420 peut être démontée de la deuxième partie vitrée 430.

Le système de retenue 400 comprend en outre un bloc support 450, une portion amovible 470 et une portion articulée 480.

Le bloc support 450 permet de positionner le système de retenue 400 dans le module 100 de mesure et en particulier de régler la position en hauteur du réservoir 410. Il comprend une portion de raccordement 460 et une base 454 formant un angle/coude avec la portion de raccordement 460, ce qui permet de faciliter la préhension par l'utilisateur.

A l'insertion du système de retenue 400 dans le module 100, la base 454 de préhension vient en butée avec le module 100, permettant à la portion de raccordement 460 et à la portion amovible 470 de s'étendre dans le module 100. La portion de raccordement 460 permet alors à son tour à la portion amovible d'être positionnée de façon adéquate dans le module 100 pour la réalisation de mesures.

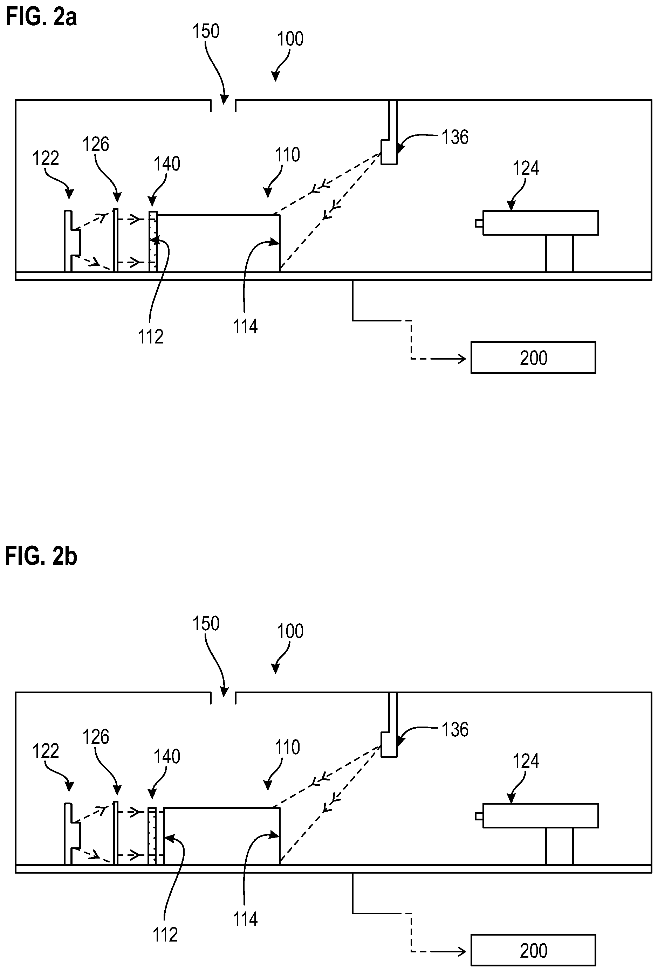

Le système de retenue 400 peut être positionné dans un logement prévu à cet effet dans le module 100 de mesure, sur le chemin optique, la base 454 pouvant alors servir de butée. En alternative, le système de retenue 400 peut être positionné au niveau d'une ouverture 150 illustrée aux figures 2a, 2b, servant à distribuer l'échantillon S. Le système de retenue 400 sert alors également d'obturateur pour empêcher que la lumière extérieure ne pénètre dans le module 100 de mesure. Dans cette configuration, le système de retenue 400 apparaîtrait suspendu tête en bas, comme illustré sur la figure 4b, la base 454 et la portion de raccordement étant agencés dans l'ouverture 150 pour prévenir tout décrochage du système de retenue 400. Lorsque le système de retenue 400 n'est pas positionné à cet endroit, un cache peut être utilisé comme obturateur de l'ouverture 150 pendant les mesures.

La portion amovible 470 comporte un logement 472 recevant le réservoir 410. Ladite portion amovible 470 est reliée de manière amovible à ladite portion de raccordement 460. Sur la figure 4a, la portion amovible 470 est illustrée en position démontée tandis que sur la figure 4b elle est illustrée en position montée. La deuxième partie vitrée 430 est fixée à la portion amovible 470 en étant immobile dans ladite portion amovible.

La portion articulée 480 comprend une ouverture ou orifice 482. La portion articulée est montée pivotante sur le bloc support 450. Lorsqu'elle pivote par rapport au bloc support 450, la portion articulée est apte à passer d'une position de montage dans laquelle elle est éloignée de la portion amovible 470 à une position d'utilisation dans laquelle elle est rabattue sur la portion amovible 470 (figure 4b). Lorsque la portion articulée 480 est en position d'utilisation, l'ouverture 482 est en vis-à-vis du réservoir 410, ce qui permet de ne pas entraver le passage du faisceau électromagnétique arrivant sur ce versant du réservoir 410, en l'occurrence le faisceau électromagnétique $E_{S1}$.

En outre, ladite portion articulée 480 comprend des moyens compressibles 486a, 486b, 488a, 488b permettant le plaquage du système de retenue 400 contre les faces intérieures du logement prévu à cet effet dans le module 100 de mesure lorsque ladite portion articulée 480 est en position d'utilisation. Ainsi, le système de retenue 400 est en contact permanent avec le logement, en particulier avec des faces intérieures dudit logement. En étant ainsi configuré, le système de retenue 400 du dispositif 1 présente un positionnement mécanique et optique identique d'une utilisation à l'autre sans que l'utilisateur ait à faire preuve d'habileté ou à effectuer un contrôle visuel rigoureux. Il permet donc d'effectuer des mesures répétables sur des échantillons variés. Cette configuration est donc préférée à celle où le système de retenue est positionné au niveau de l'ouverture 150.

En tout état de cause, l'échantillon présente un positionnement fixe au sein du module 100 de mesure.

À ce propos, l'échantillon S dont on veut déterminer les propriétés est solide. Comme cela a déjà été mentionné, il s'agit plus précisément d'un échantillon de grains, de poudres, de pâtes, et de manière générale les produits qui sont fabriqués dans l'industrie céréalière ou laitière. L'échantillon peut être de toute taille, la seule limite étant les dimensions du réservoir 110. Cela étant dit, il est préférable que la taille de l'échantillon soit adaptée pour que la quantité d'échantillon prélevée soit représentative de l'ensemble dont il a été extrait. L'échantillon est hétérogène tant dans la taille de ses éléments que dans leur forme. Il peut éventuellement contenir des impuretés et de manière générale tout corps étranger qui n'est pas à proprement parler l'élément prépondérant au sein de l'échantillon. De manière classique, tout l'enjeu de mesures par spectroscopie infrarouge et spectroscopie de fluorescence peut d'ailleurs résider dans le fait de déterminer les proportions de tels corps étrangers de manière à évaluer la qualité de l'échantillon S analysé. L'échantillon peut également être broyé au préalable comme pour des chips ou des biscuits.

Revenons à la figure 1a. Le premier sous-ensemble 120 de spectroscopie infrarouge comprend une première source 122 d'excitation, un élément optique 140 et un premier moyen 124 d'acquisition de spectres de transmittance $S_{iR}$, $S_i$ de l'échantillon S. Comme cela a été mentionné précédemment, ces éléments ne sont pas tous regroupés dans un emplacement distinct de l'emplacement des éléments composant le deuxième sous-ensemble de spectroscopie de fluorescence mais agencés de manière optimale avec ces derniers.

La première source 122 d'excitation est configurée pour émettre un rayonnement électromagnétique $E_{S1}$ dans le domaine de l'infrarouge. Elle peut également émettre dans le proche infrarouge. Elle peut, de préférence, émettre un rayonnement électromagnétique $E_{S1}$ polychromatique et à spectre large dans une gamme de longueurs d'onde comprises entre 700 et 1100 nm.

La première source 122 d'excitation émet le rayonnement électromagnétique $E_{S1}$, sous forme d'un faisceau, vers la première paroi 112 du réservoir. Le faisceau électromagnétique $E_{S1}$ se propage le long d'un axe optique X passant par la première source 122 d'excitation et centré sur ladite première source 122. Comme la première paroi 112 est transparente pour le rayonnement électromagnétique $E_{S1}$, ce dernier est apte à traverser ladite première paroi 112 et donc à éclairer l'intérieur du réservoir 110 et l'échantillon S, le cas échéant. De préférence, la première source 122 éclaire de manière homogène et collimatée la paroi 112. On entend par « collimatée » le fait que la lumière de la première source présente des rayonnements sensiblement parallèles, i.e. qui se déploient sans se disperser avec la distance.

À titre d'exemple, une source 122 d'excitation convenant à la mise en œuvre de l'invention est une source à incandescence halogène haute puissance et large bande. Outre sa capacité à éclairer dans le domaine infrarouge, ce type de source lumineuse présente une inertie élevée, ce qui permet de limiter voire supprimer les effets de scintillement dus aux fluctuations du courant d'entrée. En variante à une source à spectre large, on pourrait également utiliser plusieurs sources polychromatiques ou monochromatiques couvrant la gamme de longueurs d'onde souhaitée.

Les interactions entre le rayonnement électromagnétique $E_{S1}$ infrarouge et l'échantillon sont de nature élastique. Elles dépendent de la nature, de la force et de l'axe des liaisons chimiques des molécules de l'échantillon S analysé. Le rayonnement électromagnétique $E_{S1}$ infrarouge n'est absorbé que si le produit scalaire dudit rayonnement électromagnétique $E_{S1}$ avec le moment dipolaire électrique induit au cours de la vibration des molécules est non nul. La spectroscopie infrarouge permet donc de fournir des informations sur la structure et la composition chimique des échantillons étudiés.

Cela étant dit, afin de pouvoir obtenir des informations quantitatives, il est d'usage en spectroscopie infrarouge d'effectuer au moins deux mesures afin de mesurer le spectre de l'échantillon sans influence des autres éléments situés sur le chemin optique, ce qui inclut la source et le détecteur. C'est également le cas dans le procédé d'analyse d'un échantillon selon l'invention. Ici, une description générale en est faite afin de mieux comprendre le rôle de l'élément optique 140. Un premier spectre de transmittance $S_{iR}$, dit spectre de référence, est acquis sans échantillon, puis un second spectre de transmittance $S_i$ est acquis avec l'échantillon. Le spectre de référence $S_{iR}$ est la mesure de la contribution de l'environnement local dans le spectre $S_i$ obtenu avec l'échantillon et doit être comparé au spectre $S_i$ obtenu avec l'échantillon afin d'extraire le signal réel seulement dû à l'échantillon.

Cependant, le rapport d'intensité entre le spectre de référence $S_{iR}$ et le spectre $S_i$ de l'échantillon est très élevé, i.e. typiquement d'environ 20000. En effet, dans des conditions d'éclairage identique en terme d'intensité, le spectre de référence $S_{iR}$ présente une transmittance très élevée tandis que le spectre $S_i$ avec l'échantillon présente une transmittance plus faible. Afin de réduire cet écart de transmittance, deux solutions naturelles ont été proposées. La première consiste à ajouter un élément absorbant sur le trajet optique entre la source et le moyen de détection lors de la mesure sans échantillon. L'élément absorbant permet d'obtenir des intensités comparables avec et sans l'échantillon mais altère le chemin optique entre les deux mesures. Une « composante » supplémentaire doit donc être prise en compte dans les spectres obtenus et éventuellement supprimée, ce qui peut s'avérer complexe. La seconde solution consiste à altérer le spectre de la source elle-même en faisant varier l'intensité lumineuse entre les deux mesures. Le problème qui se pose alors est celui de la répétabilité des mesures.

L'élément optique 140 du dispositif 1 d'analyse de l'invention permet de s'affranchir de telles contraintes. En effet, l'élément optique 140 est diffusant et transparent pour le rayonnement électromagnétique $E_{S1}$ infrarouge émis par la source 122 d'excitation. La transparence de l'élément optique 140 vis-à-vis du rayonnement $E_{S1}$ lui permet de ne pas entraver la propagation dudit rayonnement $E_{S1}$. Le caractère diffusant de l'élément optique 140 permet quant à lui de dévier le rayonnement électromagnétique $E_{S1}$ dans des directions diverses et permet donc de réduire l'intensité du signal lumineux arrivant sur le premier moyen 124 d'acquisition, notamment en l'absence d'échantillon S dans le réservoir 110. En l'espèce, le phénomène de diffusion dont il s'agit est la diffusion de Rayleigh. Les caractères transparent et diffusant de l'élément optique 140 impliquent un certain nombre de phénomènes qui n'ont pas la même portée selon que le réservoir 110 comprend ou non un échantillon.

Selon un premier mode de réalisation illustré aux figures 1a et 2a, l'élément optique 140 est positionné entre la première source 122 d'excitation et la première paroi 112 du réservoir, sur le chemin optique entre la première source 122 et le premier moyen 124 d'acquisition. Autrement dit, l'élément optique 140 est situé à la fois en vis-à-vis de la première source 122 d'excitation et de la première paroi 112 du réservoir, sans toutefois être nécessairement à proximité de ladite première source 122 et ladite première paroi 112 du réservoir. Dans ce mode de réalisation de l'invention, l'élément optique 140 constitue plus précisément la première paroi 112 du réservoir. Autrement dit, la première paroi 112 et l'élément optique 140 ne forment qu'un. Encore autrement dit, l'élément optique 140 est intégral avec le réservoir 110. Ainsi, l'élément optique 140 étant localisé sur le chemin optique du rayonnement électromagnétique $E_{S_1}$, il est positionné de sorte à interagir avec celui-ci avant qu'il n'atteigne le premier moyen 124 d'acquisition.

En l'absence d'échantillon S dans le réservoir 110, le faisceau $E_{S_1}$ est amené à traverser successivement l'élément optique 140/la première paroi 112, puis la deuxième 114 paroi du réservoir avant d'atteindre le premier moyen 124 d'acquisition. En traversant l'élément optique 140, il ne peut que se propager dans de multiples directions et atteindre le premier moyen 124 d'acquisition avec une intensité réduite sans perte d'information. En présence de l'échantillon S dans le réservoir 110, le faisceau $E_{S_1}$ est amené à traverser successivement l'élément optique 140/la première paroi 112, puis l'échantillon S et enfin la deuxième paroi 114 avant d'atteindre le deuxième moyen 124 d'acquisition. En conséquence de quoi, même si le faisceau est rendu diffus à cause de l'élément optique 140, cette diffusion est négligeable par rapport à la diffusion naturellement générée par l'échantillon lorsque celui-ci est traversé par le faisceau $E_{S_1}$. Le caractère négligeable de la diffusion induite par l'élément optique 140 lors de la mesure avec échantillon dépend de l'échantillon S analysé. Si un échantillon S de grains ou de poudre est naturellement très diffusant et donc plus diffusant que l'élément optique 140, ce n'est pas nécessairement le cas pour tous les autres types d'échantillon. Le signal mesuré avec l'échantillon S en présence de l'élément optique 140 n'est donc pas de plus faible qualité comparativement au signal qui aurait été mesuré en l'absence de l'élément optique 140. En somme, l'élément optique 140 rend possible l'acquisition du spectre de référence $S_{iR}$ et du spectre $S_i$ de l'échantillon avec la première source 122 d'excitation éclairant dans les mêmes conditions lors de l'acquisition des deux spectres. Cela permet de laisser libre le choix du moyen d'acquisition.

L'élément optique 140 n'est pas nécessairement positionné parallèlement à la première paroi 112, comme cela est illustré sur les figures. De préférence, l'élément optique 140 génère une diffusion isotrope, en l'occurrence dans toutes les directions. Ainsi, dans la mesure où il est positionné sur le chemin optique, il peut être incliné par rapport à l'axe optique X sans empêcher la propagation du faisceau électromagnétique $ES_1$ infrarouge selon l'axe optique X, tout en exerçant sa fonction première qui est de le rendre diffus. Par exemple, l'élément optique 140 peut être fabriqué par dépolissage d'un verre.

Selon un deuxième mode de réalisation illustré aux figures 1b et 2b, l'élément optique 140 et la première paroi 112 du réservoir peuvent être séparés. Dans cette configuration, l'élément optique 140 se présente sous la forme d'un élément distinct de la première paroi 112. Il peut éventuellement se situer à distance du réservoir 110 et être stabilisé au moyen d'un support, mais ce n'est pas obligatoire. Il peut également être collé à la première paroi 112 du réservoir, sans être ladite première paroi 112. Quelle que soit la configuration envisagée, i.e. que l'élément optique 140 constitue la première paroi 112 ou en soit séparé, ce qui importe dans le cadre de l'invention est que l'élément optique 140 soit localisé sur le chemin optique du faisceau électromagnétique $E_{S_1}$ avant que celui-ci n'atteigne le premier moyen 124 d'acquisition.

Le premier sous-ensemble 120 peut comprendre, outre l'élément optique 140, une lentille de collimation 126 pour la source 122 d'excitation située sur le chemin optique du faisceau électromagnétique $E_{S_1}$. De préférence, la lentille de collimation 126 est située entre la source 122 d'excitation et l'élément optique 140, que ce dernier soit intégral avec le réservoir 110 ou séparé de celui-ci. La lentille de collimation 126 permet de rendre parallèle le faisceau électromagnétique $E_{S_1}$ émanant de la source 122 d'excitation de sorte que l'intérieur du réservoir 110, en particulier l'échantillon S, soit éclairé de manière homogène. Les informations obtenues à partir de la mesure sont d'autant plus qualitatives et quantitatives.

Dans une variante de réalisation des premier et deuxième modes de réalisation de l'invention, l'élément optique 140 pourrait à la fois permettre de rendre diffus le faisceau électromagnétique $E_{S_1}$ et en même temps le collimater. Dans ce cas, la lentille de collimation 126 n'est pas nécessaire puisque l'élément optique 140 joue le rôle de ladite lentille de collimation en remplissant sa fonction première qui est de rendre diffus les rayonnements venant de la première source 122 d'excitation. Par exemple, un tel élément peut être fabriqué par dépolissage d'une lentille de collimation.

En outre, comme mentionné précédemment, le premier sous-ensemble 120 comprend un premier moyen 124 d'acquisition de spectres de transmittance. À cet égard, le premier moyen 124 d'acquisition permet de détecter un signal électromagnétique émis dans les domaines visible, proche-infrarouge et infrarouge notamment à des longueurs d'onde comprises entre 750 et 2500 nm. Comme cela a été vu dans les sections qui précèdent, l'utilisation de l'élément optique 140 laisse le choix du moyen d'acquisition libre.

Dans un mode de réalisation préféré de l'invention, on peut avoir recours pour ce premier moyen 124 d'acquisition à un unique détecteur à transfert de charge ou en référence à la terminologie anglaise (*Charged Coupled Device* (CCD) en anglais) capteur CCD. Il est également possible d'utiliser un détecteur à base de capteurs CMOS (*Complementary Metal Oxide Semiconductor*), à base de photodiodes ou tout autre moyen de détection connu de l'homme de l'art. En pratique, il est préférable d'utiliser le détecteur avec un monochromateur. Le monochromateur permet de sélectionner le(s) domaine(s) spectral(aux) souhaité(s), c'est-à-dire adapter la collection du signal à l'analyse envisagée. Les monochromateurs envisageables sont par exemple un(des) filtre(s) chromatique(s) ou encore un spectrographe.

Le premier moyen 124 d'acquisition est avantageusement aligné avec la source 122 d'excitation, l'élément optique 140 diffusant et transparent et le réservoir 110 le long de l'axe optique X. Autrement dit, ces éléments sont tous sur le chemin optique. Le premier moyen 124 d'acquisition présente un angle de champ centré autour de l'axe optique X. Pour autant, ce qui importe ici est que le premier moyen 124 d'acquisition est agencé de manière à détecter le(s) signal(aux) de références $S_{iR}$ et le(s) signal(aux) $S_i$ avec l'échantillon émis sous éclairement du faisceau infrarouge $E_{S1}$.

Si du point de vue du sous-ensemble 120 de spectroscopie infrarouge, les agencements tels que précédemment décrits permettant de réaliser des mesures de spectroscopie infrarouge sont ingénieux en ce qu'ils prévoient l'élément optique 140 diffusant et transparent, ils le sont encore plus en ce qu'ils n'entravent pas les mesures par spectroscopie de fluorescence réalisées sur le même échantillon S. Cela est plus précisément décrit dans la suite.

Le deuxième sous-ensemble 130 de spectroscopie de fluorescence comprend une deuxième source 132 d'excitation et un deuxième moyen 134 d'acquisition de signaux de fluorescence $S_{f1}$, $S_{f2}$ dudit échantillon S.

La deuxième source 132 d'excitation est configurée pour émettre un rayonnement électromagnétique $E_{S2}$ dans le domaine ultraviolet. Elle peut en outre émettre dans le domaine visible. Selon un mode préférentiel de réalisation, elle peut émettre un rayonnement monochromatique ayant une longueur d'onde comprise entre 250 et 550 nm. Un exemple de deuxième source 132 d'excitation pouvant être utilisée dans le dispositif 1 d'analyse selon l'invention consiste en au moins une diode électroluminescente (*Light Emitting Diodes (LED)* en anglais) émettant à une longueur d'onde de 280 nm, 340 nm, 385 nm ou 420 nm. L'avantage des sources LEDs est leur capacité à éclairer de manière intense et homogène. En outre, elles présentent une longue durée de vie.

De préférence, le nombre de sources 132 d'excitation peut être adapté selon la taille de la surface à analyser. Lorsque plusieurs deuxièmes sources 132 d'excitation sont utilisées, on peut alors prévoir un support 136 comme représenté sur les figures 1a et 1b configurés pour accueillir les sources 132 et ainsi maintenir les sources sur un support unique. À cet égard, dans un exemple de réalisation illustré sur la figure 3, le support 136 comprend une portion 1360 d'assemblage et une portion une portion d'appui (non illustrée) permettant de stabiliser la portion 1360 d'assemblage. La portion 1360 d'assemblage comprend une pluralité de logements 1362 disposés en cercle autour d'une ouverture 1364 centrale du support et dans lesquelles les deuxièmes sources 132 sont aptes à être fixées. En effet, les logements 1362 présentent des dimensions appropriées pour recevoir les deuxièmes sources 132 et comportent des moyens de fixations des sources 132 permettant le maintien des sources par exemple au moyen de vis et/ou d'écrous. Préférentiellement, la portion 1360 d'assemblage est amovible de sorte à pouvoir être retirée du support 136.

En variante, on pourrait également utiliser comme deuxième source 132 d'excitation une source à spectre large émettant un rayonnement électromagnétique $E_{S2}$ dans le domaine ultraviolet et un monochromateur en combinaison avec une telle source. Dans cette configuration, la source à spectre large étant de nature polychromatique, il est nécessaire de l'associer à un monochromateur afin de sélectionner une gamme plus étroite de longueurs d'onde ou une longueur d'onde à partir du faisceau électromagnétique $E_{S2}$ de spectre plus large. Cette configuration est plus complexe que la précédente, i.e. avec les sources LEDs. Un exemple de source à spectre large émettant dans le domaine ultraviolet est une lampe au deutérium qui émet dans un domaine ultraviolet à des longueurs d'onde comprises entre 180 nm et 370 nm.

La deuxième source 132 d'excitation émet le faisceau électromagnétique $E_{S2}$ vers la deuxième paroi 114 du réservoir, du côté opposé du réservoir 110 où est localisé l'élément optique 140 diffusant et transparent selon les premier et deuxième modes de réalisation (figures 1a, 1b,2a, 2b). La deuxième source 132 d'excitation peut être excentrée par rapport l'axe optique X. Lorsque plusieurs sources 132 sont utilisées, chacune des sources peut être excentrée par rapport audit axe optique X comme cela est illustré sur les figures 4a à 4c. La(es) source(s) 132 sont avantageusement inclinées en direction du centre de la deuxième paroi 114, ce qui permet un éclairage homogène et approprié de ladite deuxième paroi 114 par lesdites sources. Ce qui importe dans le cas présent est que les deuxièmes sources 132 soient positionnées de sorte à ne pas empêcher l'acquisition des spectres infrarouge et de fluorescence.

Le fait que la deuxième source 132 émette le faisceau électromagnétique $E_{S2}$ vers la deuxième paroi 114 n'est ni anecdotique ni un simple choix d'agencement. Comme cela a été mentionné au préalable de la description détaillée de l'invention, il est question dans la présente invention de fournir un dispositif 1 permettant de réaliser des mesures de spectroscopie infrarouge et de spectroscopie de fluorescence en temps réduit sur le même échantillon S sans avoir à effectuer les mesures dans des sous-modules séparés, et donc sans avoir à séparer l'échantillon en deux ou encore avoir à le transporter d'un module à l'autre. Ce choix d'agencement permet d'effectuer les mesures de spectroscopie infrarouge et de spectroscopie de fluorescence sur le même échantillon sans que les éléments du premier sous-ensemble 120, nécessaires aux mesures de spectroscopie infrarouge, ne perturbent les mesures de spectroscopie de fluorescence.

En effet, la très faible intensité de la fluorescence au regard de l'intensité de la source la rend très sensible aux phénomènes de diffusion, la diffusion étant elle-même dépendante de la nature physico-chimique de l'échantillon. Une diffusion, même de faible intensité, perturbe, c'est-à-dire parasite le signal de fluorescence. Si elle n'empêche pas d'en extraire des données quantitatives, la composante due à la diffusion des spectres complexifie de manière significative l'extraction des données utiles pour l'analyse et/ou requiert l'utilisation de filtres qui à l'heure actuelle ont un effet très limité sur la réduction de la part de lumière due à la diffusion dans le spectre final.

Du fait de son positionnement entre la première source 122 d'excitation et la première paroi 112 du réservoir, l'élément optique 140 diffusant peut jouer son rôle pour les mesures de spectroscopie infrarouge sans gêner les mesures par spectroscopie de fluorescence. En effet, les mesures par spectroscopie de fluorescence ne requièrent qu'un éclairage du réservoir 110, le cas échéant de l'échantillon S du côté de la deuxième paroi 114, c'est-à-dire du côté opposé à l'élément optique 140 diffusant. Aucune interaction ne peut donc se produire entre le faisceau électromagnétique $E_{S2}$ émis par la deuxième source 132 d'excitation et l'élément optique 140. L'agencement ingénieux des éléments du module 100 et les directions optimisées d'éclairage du réservoir 110 et de l'échantillon S avec les première 122 et deuxième 132 sources d'excitation permettent d'effectuer les deux types de mesures sans que l'une « parasite » l'autre. Ainsi, l'enjeu de l'invention qui est de pouvoir effectuer les mesures de spectroscopie infrarouge et, dans un délai réduit de pouvoir effectuer les mesures de spectroscopie de fluorescence sur le même échantillon est atteint. Cet agencement permet aussi que le deuxième sous-ensemble 130, nécessaire aux mesures de spectroscopie de fluorescence, ne perturbe pas les mesures de spectroscopie infrarouge.

Il convient de noter que si l'élément optique 140 diffusant est situé entre la première source 122 d'excitation et la première paroi 112 du réservoir dans les premier et deuxième mode de réalisation de l'invention, un autre positionnement peut être envisagé par l'homme du métier, tout en restant dans le concept inventif de l'invention, dès lors que ledit élément optique 140 est positionné de manière à ne pas entraver la mesure par spectroscopie de fluorescence et qu'il est positionné entre la première source 122 d'excitation et le premier moyen 124 d'acquisition. L'élément optique 140 est positionné de sorte à ne pas entraver les mesures par spectroscopie de fluorescence dès lors qu'il ne perturbe pas le faisceau électromagnétique $ES_2$ émis par le deuxième moyen 134 d'acquisition et qu'il ne perturbe pas le signal de fluorescence émis par l'échantillon lorsqu'il est exposé à un tel faisceau. L'élément optique 140 est positionné entre la première source 122 et le premier moyen d'acquisition 124 dès lors qu'il est positionné de sorte à rendre diffus le rayonnement $E_{S1}$ émis par ladite première source 122 sans empêcher sa détection par le premier moyen 124 d'acquisition lors des mesures avec et sans échantillon, en tenant compte des contraintes précitées.

Ainsi, dans un autre mode de réalisation de l'invention, l'élément optique 140 peut aussi être positionné entre la deuxième paroi du réservoir 114 et le premier moyen 124 d'acquisition, hors d'un trajet optique du rayonnement électromagnétique $E_{S2}$ émis par la deuxième source 132 d'excitation et hors d'un angle solide de collecte d'un signal de fluorescence émis par l'échantillon S lorsqu'il est exposé rayonnement électromagnétique $E_{S2}$ émis par la deuxième source 132 d'excitation. Dans ce mode de réalisation, la deuxième paroi 114 du réservoir ne peut pas être constituée de l'élément optique 140 puisque cela gênerait nécessairement la mesure par spectroscopie de fluorescence. Un exemple de dispositif 100 correspondant à une telle mise en œuvre est illustré à la figure 1c.

En outre, la deuxième paroi 114 du réservoir est transparente pour le rayonnement électromagnétique $E_{S2}$ émis par la deuxième source 132, de sorte que ledit rayonnement $E_{S2}$ est apte à traverser la deuxième paroi 114 et donc à éclairer l'intérieur du réservoir 110 et l'échantillon S. En outre, la deuxième paroi 114 du réservoir est anti-réfléchissante pour le rayonnement électromagnétique $E_{S2}$. Le caractère anti-réfléchissant de la deuxième paroi 114 permet d'augmenter la part de la lumière qui est transmise à travers la deuxième paroi 114 et qui atteint l'échantillon S. Cela permet donc d'améliorer la détection du signal de fluorescence qui peut s'avérer relativement faible et permet donc d'optimiser les mesures par spectroscopie de fluorescence.

En outre, la deuxième paroi 114 du réservoir est également transparente pour le rayonnement électromagnétique $E_{S1}$ infrarouge émis par la première source 122 d'excitation et pour le rayonnement électromagnétique infrarouge réémis par l'échantillon S ou l'environnement avoisinant celui-ci.

À ce propos, comme cela a été mentionné précédemment, le support 136 comprend également une ouverture 1364 centrale. L'ouverture 1364 est centrale en ce qu'elle laisse une large zone vide dépourvue de matière autour de l'axe optique X, ce qui permet de laisser passer le rayonnement électromagnétique généré par les mesures de spectroscopie infrarouge et spectroscopie de fluorescence. L'ouverture 1364 est de taille appropriée pour laisser passer le plus de rayons possibles, ce qui permet de collecter un maximum de signal pour la mesure par spectroscopie infrarouge. Dans l'exemple de réalisation illustré, l'ouverture 1364 centrale est de forme circulaire, mais cela n'est pas obligatoire. L'ouverture 1364 centrale peut adopter toute autre forme dès lors qu'elle n'entrave pas la collecte du signal infrarouge.

Le deuxième moyen 134 d'acquisition est dédié à la mesure par spectroscopie de fluorescence. À cet égard, le deuxième moyen 134 d'acquisition permet de détecter le signal électromagnétique émis dans les domaines ultraviolet et visible, et plus particulièrement à des longueurs d'onde comprises entre 200 et 550 nm. Le deuxième moyen 134 d'acquisition peut être un capteur CCD, un capteur CMOS, une photodiode ou tout autre moyen de détection connu de l'homme de l'art. Il est préférable d'utiliser le détecteur avec un monochromateur, par exemple un(des) filtre(s) chromatique(s) ou encore un spectrographe.

Le deuxième moyen 134 d'acquisition fait avantageusement un angle $\alpha$ avec l'axe optique X. Autrement dit, un axe X' passant ledit deuxième moyen 134 d'acquisition et un plan médian et sensiblement orthogonal à la deuxième paroi 114 du réservoir fait un angle $\alpha$ avec l'axe optique X. L'axe X' est donc l'axe que présente le deuxième moyen 134 d'acquisition par rapport à l'axe optique X. Ainsi, le deuxième moyen 134 n'est pas aligné optiquement avec les autres éléments du module 100, à savoir, la première source 122 d'excitation, l'élément optique 140, le réservoir 110, la deuxième source 132 d'excitation et le premier moyen 124 d'acquisition. Il en résulte que bien que la deuxième source 132 émette un rayonnement électromagnétique $E_{S2}$ de part et d'autre de l'axe optique X, seule la portion de ce rayonnement électromagnétique localisée dans un angle de champ du deuxième moyen 134 d'acquisition autour de la direction d'axe X' est détectable.

L'éclairage de la deuxième paroi 114 par la deuxième source 132 génère de la réflexion spéculaire qui reste significatif malgré un traitement anti-réfléchissant de la deuxième paroi 114 du réservoir. La réflexion est dite spéculaire lorsque le rayonnement incident est réfléchi selon une direction donnée à la manière d'un faisceau réfléchi par un miroir. Elle peut causer la saturation du moyen de détection puisqu'il existe toujours une part importante du rayonnement électromagnétique qui est réfléchie de manière spéculaire dans la direction de l'axe optique X. Dans le cas d'espèce, la deuxième source 132 d'excitation émet le rayonnement électromagnétique $E_{S2}$ vers la deuxième paroi 114 qui est traversée par ledit axe optique X, ce qui génère nécessairement de la réflexion spéculaire. L'angle $\alpha$ que fait le deuxième moyen 134 d'acquisition avec l'axe optique X est choisi de sorte que ledit second moyen 134 d'acquisition n'est pas directement positionné sur le trajet optique des rayons réfléchis de manière spéculaire par la deuxième paroi 114 du réservoir, ce qui permet d'éviter la saturation dudit deuxième moyen 134 d'acquisition, tout en gardant le maximum de fluorescence émise de manière isotrope par l'échantillon. Par exemple, l'angle $\alpha$ prend une valeur égale à 10°.

Le module 100 de mesure tel que précédemment décrit permet de réunir en un unique module les éléments permettant d'effectuer à la fois des mesures de spectroscopie infrarouge et de spectroscopie de fluorescence sans avoir à séparer l'échantillon en deux portions, et donc d'avoir à faire deux analyses séparées spatialement voire même peut être temporellement, ou encore sans avoir à réaliser les deux types de mesure successivement dans le temps. Si le dispositif 1 selon l'invention permet d'effectuer les mesures par les deux types de spectroscopie sur le même échantillon S dans un délai de quelques minutes, il est également configuré pour traiter les données qui en résultent.

À cet égard, le dispositif 1 selon l'invention comprend en plus du module 100 de mesure, un module 200 de traitement connecté au module 100 de mesure. Le module 200 de traitement peut être tout type de moyen de traitement électronique ou informatique, par exemple un ordinateur, un smartphone ou tout appareil similaire à une borne avec un écran de contrôle, une clé USB, une carte mémoire mobile ou tout autre technologie similaire. De préférence, le module 200 de traitement est un PC embarqué.

Le module 200 de traitement est connecté à l'unique module 100 de mesure du dispositif 1 d'analyse au moyen d'un réseau 300 de communication. Le réseau 300 de communication permet de connecter le module 200 de traitement au module 100 de mesure. Par exemple, le réseau 300 de communication est un réseau local tel qu'un réseau filaire, un réseau bluetooth, un réseau wifi ou encore un réseau éthernet. Dans tous les cas, le réseau 300 de communication est configuré pour transmettre des informations entre le module 200 de traitement et le module 100 de mesure du dispositif 1. Du fait que le module 100 de mesure est unique, il n'y a donc qu'une interface entre la mesure et le traitement.

Le module 200 de traitement comprend un processeur 220 et une mémoire 240.

La mémoire 240 est configurée pour recevoir et stocker les données transmises par le réseau 300 de communication. Ces données peuvent comprendre tout type d'information mesuré par le module 100 de mesure tel que des longueurs d'onde des rayonnements infrarouge $E_{S1}$ et de fluorescence $E_{S2}$ émis par l'échantillon, des intensités mesurées de ces rayonnements ou encore des spectres électromagnétiques correspondants.

Le processeur 220 est configuré pour analyser et/ou traiter les données obtenues par spectroscopie infrarouge et spectroscopie de fluorescence. À cet égard, un logiciel de traitement de ces données peut être installé sur le processeur afin d'automatiser le traitement de ces données en temps réel. Ce qui importe dans le cadre de la présente invention est que le processeur 220 soit en mesure de traiter les données venant des mesures de spectroscopie infrarouge et celles venant des mesures de spectroscopie de fluorescence séquentiellement et dans un délai de quelques minutes pour en extraire des facteurs et/ou critères d'appréciation de l'échantillon S étudié. Les opérations effectuées par le processeur 220 seront décrites plus en détail dans la suite en relation avec le procédé d'analyse d'un échantillon S.

En effet, l'invention concerne en outre un procédé d'analyse d'un échantillon S mis en œuvre au moyen d'un dispositif 1 d'analyse tel que précédemment décrit. Le procédé selon l'invention comprend les étapes suivantes.

Lors d'une première étape A), on acquiert un spectre de transmittance $S_i$ de l'échantillon S au moyen dudit premier sous-ensemble 120 de spectroscopie infrarouge. Si en théorie, il est possible de réaliser une telle mesure directement, il n'est pas possible de quantifier avec précision le phénomène propre à l'échantillon, i.e. transmittance de l'échantillon, sans une mesure supplémentaire du spectre infrarouge de référence $S_{iR}$ dit autrement spectre sans échantillon. Comme cela a été décrit précédemment, la mesure du spectre de référence $S_{iR}$ permet de quantifier la contribution de l'environnement local dans la mesure du spectre de transmittance de l'échantillon.

L'étape A) du procédé selon l'invention comprend avantageusement des sous-étapes Aa), Ab), Ac), Ad) et Ae), les étapes Aa) et Ab) étant relatives à la mesure du spectre de référence $S_{iR}$ et sont donc réalisées sans l'échantillon à analyser tandis que les étapes Ac) à Ae) concernent la mesure du spectre $S_i$ de l'échantillon et sont donc mises en œuvre avec l'échantillon à analyser.

Lors de la sous-étape Aa), on éclaire le réservoir 110 au moyen d'un rayonnement électromagnétique $E_{S1}$ généré par la première source 122. Le faisceau électromagnétique $E_{S1}$ traverse l'élément optique 140, puis la première paroi 112, l'intérieur du réservoir et la deuxième 114 paroi du réservoir, dans cet ordre, avant d'atteindre le premier moyen 124 d'acquisition. Le faisceau électromagnétique $E_{S1}$ décrit ainsi une trajectoire selon l'axe optique X. L'intérieur du réservoir étant vide, il ne comprend pas d'échantillon. Pour autant, le réservoir 110 reste rempli d'air et autres éléments présents dans l'air qui sont susceptibles d'absorber et réfléchir une partie du rayonnement électromagnétique $E_{S1}$.

Précisons que si la première paroi 112 du réservoir est constituée par l'élément optique 140, le faisceau $E_{S1}$ traversera donc successivement l'élément optique 140, l'intérieur du réservoir et la deuxième paroi 114 du réservoir avant d'atteindre le premier moyen 124 d'acquisition. Précisons également, que si une lentille de collimation 126 est optionnellement interposée entre la première source 122 d'excitation et l'élément optique 140, l'ordre de passage à travers les différents éléments en sera concomitamment affecté. Précisons encore que dans un mode de réalisation où la deuxième source 132 d'excitation comprend une pluralité de sources supportées par le support 136, le faisceau électromagnétique $E_{S1}$ traverse nécessairement l'ouverture 1364 dudit support qui est positionnée sur le chemin optique.

Lors de la sous-étape Ab), on mesure le spectre de transmittance $S_{iR}$ de référence.

Lors de la sous-étape Ac), on remplit le réservoir 110 de l'échantillon S à étudier. Si des mesures comparatives doivent être effectuées entre plusieurs échantillons, on prendra soin de remplir systématiquement le réservoir 110 avec la même quantité d'échantillon.

Lors des sous-étapes Ad) et Ae), on reproduit les étapes Aa) et Ab) à la différence que dans ce cas l'échantillon S à étudier est placé à l'intérieur du réservoir 110. À la fin de cette seconde séquence d'acquisition, on obtient le spectre de transmittance $S_i$ de l'échantillon.

Les données collectées lors de l'étape A), notamment les spectres de transmittance de référence $S_{iR}$ et $S_i$ de l'échantillon sont ensuite analysées et/ou traitées par le processeur 220. De manière préférentielle, elles sont stockées dans la mémoire 240 du module de traitement. Nous y reviendrons dans la suite.

Lors d'une étape B du procédé selon l'invention, on acquiert des spectres de fluorescence $S_{f1}$, $S_{f2}$ dudit échantillon S au moyen du deuxième sous-ensemble 130 de spectroscopie de fluorescence. L'échantillon S dont il s'agit est rigoureusement le même échantillon analysé lors de l'étape A). Autrement dit, il ne s'agit pas d'un échantillon qui aurait été divisé pour effectuer les deux types de mesure. D'ailleurs il convient de préciser que l'étape B) n'est pas nécessairement mise en œuvre après l'étape A). Elle peut être réalisée indifféremment avant ou après l'étape A).

L'étape B) du procédé comprend des sous-étapes Ba), Bb), Bc) et Bd) qui sont décrites dans ce qui suit. Avec un temps d'intégration constant, lorsqu'on effectue la mesure par spectroscopie de fluorescence sur une gamme de longueurs d'onde étendue, par exemple comprise entre 250 nm et 650 nm, l'intensité du signal peut être exploitable sur une partie de cette gamme de longueurs d'onde tandis qu'elle peut ne pas être exploitable sur une autre partie de cette gamme de longueurs d'onde et saturer le moyen d'acquisition. Cela vient de la gamme dynamique entre la diffusion et la fluorescence. En effet, un spectre de fluorescence comprend toujours une gamme de longueurs d'onde sur laquelle on mesure de la diffusion et une gamme de longueurs d'onde sur laquelle on mesure le signal de fluorescence proprement dit. Cependant, l'intensité maximale du signal mesuré venant de la diffusion est bien plus élevée que celle venant du phénomène de fluorescence. Le rapport d'intensité mesuré entre les deux mesures peut être environ égal à 100. Or, ces deux composantes du signal sont d'égale importance aux fins de l'analyse physico-chimique de l'échantillon. Il est donc souhaitable de les ramener à des niveaux d'intensité comparable.

Lors d'une première sous-étape Ba), on éclaire le réservoir 110 et l'échantillon S au moyen du rayonnement électromagnétique $E_{S2}$ émis par la deuxième source 132 d'excitation. Compte-tenu de l'agencement des éléments du deuxième sous-ensemble 130, le faisceau électromagnétique $E_{S2}$ émis par la première source 132 traverse la deuxième paroi 114 du réservoir puis l'échantillon dans cet ordre. L'interaction du faisceau électromagnétique $E_{S2}$ avec l'échantillon S génère alors un signal de fluorescence spécifique audit échantillon S.

Lors d'une deuxième sous-étape Bb), on acquiert simultanément avec l'étape Ba), un premier spectre de fluorescence $S_{f1}$ dudit échantillon S au moyen du deuxième moyen 134 d'acquisition avec un temps d'intégration $t_1$ prédéterminé. La détermination de ce temps d'intégration peut nécessiter des mesures préalables selon l'échantillon S à étudier afin d'optimiser soit la part de signal due à la diffusion soit la part de signal due à la fluorescence. Le signal est considéré comme optimisé lorsque le rapport signal sur bruit est suffisamment élevé pour permettre l'extraction des paramètres souhaités. Quoiqu'il en soit et comme cela a été mentionné dans les sections qui précèdent, le signal mesuré par le deuxième moyen 134 d'acquisition peut, compte-tenu de la contribution de ces deux phénomènes, i.e. diffusion et fluorescence, dans le spectre final se révéler inexploitable sur une partie de cette gamme de longueurs d'onde.

Lors d'une troisième sous-étape Bc) et une quatrième sous-étape Bd), on reproduit les sous-étapes Ba) et Bb) mais cette fois-ci en sélectionnant un temps d'intégration $t_2$ optimisé pour mesurer l'autre composante du signal, c'est-à-dire soit la part de signal due à la diffusion soit la part de signal due à la fluorescence pour lequel le temps d'intégration n'avait pas été optimisé lors de la sous-étape Bb). À l'issu de ces étapes, on obtient un deuxième spectre de fluorescence $S_{f2}$.

Considérons, par exemple, que l'on cherche à mesurer le signal de fluorescence sur une gamme de longueurs d'onde comprise entre $\lambda_1$ et $\lambda_n$. Avec le temps d'intégration $t_1$, le signal de fluorescence était inexploitable sur une gamme $\lambda_1$ à $\lambda_m$ bien qu'étant exploitable sur le reste de la gamme, i.e. $\lambda_{m+i}$ à $\lambda_n$, i étant le pas entre les mesures. Lors de la séquence d'acquisition de $S_{f2}$, on choisira un temps d'intégration $t_2$ supérieur à $t_1$ si le motif pour lequel le signal de fluorescence était inexploitable sur la gamme $\lambda_1$ à $\lambda_m$ est un faible rapport signal sur bruit ou, au contraire, on choisira un temps d'intégration $t_2$ inférieur à $t_1$ si le motif pour lequel le signal de fluorescence était inexploitable sur la gamme $\lambda_1$ à $\lambda_m$ est une saturation du deuxième moyen 134 d'acquisition.

Ainsi, à la fin de la deuxième étape B) du procédé selon l'invention, on obtient deux spectres de fluorescence $S_{f1}$ et $S_{f2}$ qui sont collectés puis traités par le processeur 220. Rappelons également que le spectre infrarouge $S_{iR}$ de référence et le spectre infrarouge $S_i$ de l'échantillon associés à la mesure par spectroscopie infrarouge sont également collectés et traités par le processeur 220.

Bien que dans le mode de réalisation précédemment décrit l'étape B) de mesures de spectroscopie de fluorescence est réalisée après l'étape A) de mesure de spectroscopie infrarouge, il est aussi possible de réaliser l'étape B) avant l'étape A) sans préjudice quelconque puisque les étapes sont indépendantes.

Lors d'une troisième étape C) du procédé selon l'invention, on analyse les données obtenues par spectroscopie infrarouge et spectroscopie de fluorescence au moyen du module de traitement 200, le processeur 220 étant configuré pour déterminer au moins un indicateur caractérisant ledit échantillon à partir des données issues de l'analyse. L'étape C) du procédé selon l'invention est une étape mise en œuvre par ordinateur. Le mot ordinateur a un sens large et désigne tout moyen équipé d'un processeur et apte à exécuter des tâches en fonction des commandes qui ont été programmées. Quoiqu'il en soit soi, à l'issue de l'étape C), on obtient au moins un indicateur caractérisant l'échantillon S étudié. À ce propos, on peut sans doute préciser que cette étape est préférablement mise en œuvre après les étapes A) et B).

Dans ce qui suit, on évoquera tour à tour le traitement des spectres infrarouge et de fluorescence obtenus à l'issus des étapes A) et B) respectivement.

Lors d'une première sous-étape Ca), on détermine un spectre de transmittance $S_{if}$ final de l'échantillon S à partir des spectres $S_{iR}$ de référence et $S_i$ de l'échantillon. À cet égard, on peut en premier lieu appliquer un lissage médian à plus ou moins un ($\pm 1$) pixel pour éliminer les pixels défectueux dans les spectres infrarouge $S_{iR}$ de référence et $S_i$ de l'échantillon. Puis dans un second temps, il s'agit de retirer la contribution de l'environnement local au spectre $S_i$ de l'échantillon. Pour cela, on calcule le rapport entre le signal $S_i$ de l'échantillon et le signal $S_{iR}$ de référence. À ce stade, il convient de noter que le rapport d'intensité entre les deux spectres est typiquement de 1000. L'utilisation de l'élément optique diffusant permet donc de diviser par 20 le rapport d'intensité entre la mesure sans échantillon $S_{iR}$ et la mesure avec échantillon $S_i$. On obtient ainsi le spectre de transmittance final $S_{if}$. On peut également effectuer une normalisation du spectre ainsi obtenu pour le ramener à des valeurs en pourcentage.

Lors d'une deuxième sous-étape Cb), on élabore un spectre de spectroscopie de fluorescence $S_{ff}$ final de l'échantillon S à partir des spectres $S_{f1}$ et $S_{f2}$ (considérant l'exemple mentionné précédemment). Ce spectre est appelé dans la suite « spectre final » $S_{ff}$. Une concaténation des spectres est effectuée en 1) récupérant le spectre optimisé du signal de diffusion dans le spectre $S_{f1}$ ou $S_{f2}$ mesuré à cet effet, 2) récupérant le spectre optimisé du signal de fluorescence dans l'autre spectre $S_{f1}$ ou $S_{f2}$ mesuré à cet effet et 3) en regroupant les spectres optimisés de diffusion et de fluorescence afin d'obtenir un spectre final $S_{ff}$ ayant un bon rapport signal sur bruit aussi bien pour les composantes spectrales dues à la diffusion que pour les composantes spectrales dues à la fluorescence. Les rapports signal-sur-bruit de la part du spectre due à la diffusion et de la part du spectre due à la fluorescence sont ainsi ramenés à une valeur de 100 sur tout le spectre, notamment sur la partie du spectre correspondant à la composante fluorescence, ce qui est bien supérieur au rapport de 10 en l'absence de traitement. Les gammes de longueurs d'onde sur lesquelles s'étendent les spectres $S_{f1}$ et $S_{f2}$ sont précisées dans la suite.

En continuant avec l'exemple précédent, on pourra alors concaténer le spectre $S_{f1}$ correspondant à une mesure optimale de la fluorescence réalisée sur une gamme de longueurs d'onde comprise entre $\lambda_{m+i}$ à $\lambda_n$ - i est un entier naturel correspondant au pas - avec le spectre $S_{f2}$ correspondant à une mesure optimale de la diffusion réalisée sur une gamme de longueurs d'onde comprise entre $\lambda_1$ à $\lambda_m$. Le spectre de fluorescence $S_{ff}$ final ainsi obtenu s'étend entre $\lambda_1$ à $\lambda_n$ et présente une bonne qualité aussi bien dans la partie du spectre associée à la part du signal issu de la diffusion que celle associée à la part du signal issu de la fluorescence. La concaténation peut également permettre de concaténer des spectres qui n'ont pu être mesurés qu'avec des sources 132 différentes, par exemple à cause des longueurs d'onde de coupure définies par les LEDs. Une dernière sous-étape consisterait à effectuer un lissage gaussien du spectre $S_{ff}$ final ainsi obtenu.

En pratique, l'un parmi les spectres $S_{f1}$ et $S_{f2}$ correspond à la composante « diffusion » du spectre de fluorescence et s'étend sur une gamme de longueurs d'onde sensiblement comprise entre 250 nm et une longueur d'onde de concaténation tandis que l'autre parmi les spectres $S_{f1}$ et $S_{f2}$ correspond à la composante « fluorescence » du spectre de fluorescence et s'étend sur une gamme de longueurs d'onde sensiblement comprise entre la longueur d'onde de concaténation et 650 nm, voire plus de 650 nm. La longueur d'onde de concaténation se situe au-delà de la longueur d'onde d'excitation (i.e. la longueur d'onde rayonnement électromagnétique $E_{S2}$) et à la valeur minimale dans une gamme de longueurs d'ondes proche de la longueur d'onde d'excitation. Dans le cas de la mesure de grains de céréales, la longueur d'onde de concaténation est égale à la longueur d'onde d'excitation à laquelle on ajoute entre 10 et 20 nm.

Grâce à ce système de mesures couplées, l'acquisition d'un spectre de transmittance infrarouge et d'un spectre d'émission de fluorescence sur le même sous-échantillon permet de réunir l'ensemble des informations spectrales apportées par ces deux technologies optiques, informations en partie complémentaires, pour enrichir la connaissance de l'échantillon hétérogène analysé. Grâce au dispositif mis en œuvre, la cohérence entre les informations acquises par les deux technologies infrarouge et fluorescence, puisque les spectres correspondent au même échantillon, permet de manière idéale, et pour la première fois, de les rassembler pour opérer une fusion des informations contenues dans chacun des spectres.

Soulignons que dans la méthode de l'art antérieur, dite méthode de référence, les troisième et quatrième sous-étapes Bc) et Bd) n'existent pas. Seul le spectre de fluorescence $S_{f1}$ est acquis avec des paramètres opératoires uniques. La conséquence étant que soit la composante du signal due à la diffusion est optimisée, soit la composante du signal due à la fluorescence est optimisée, soit aucune des deux n'est optimisée. Ainsi, selon la méthode de référence, le spectre de fluorescence final $S_f$ correspond au spectre $S_{f1}$ puisqu'aucun spectre $S_{f2}$ n'est acquis.

En plus, de la méthode de concaténation des spectres vue précédemment, d'autres méthodes peuvent être mises en œuvre pour exploiter l'information de ces spectres de fluorescence et infrarouge, plusieurs méthodes sont possibles.

Mais avant toute chose, les spectres de transmittance infrarouge et d'émission de fluorescence doivent dans un premier temps être traités puis fusionnés. Il s'agit d'un prétraitement.

Les spectres de fluorescence sont traités pour isoler la diffusion du spectre de fluorescence et obtenir ainsi un spectre de fluorescence pure. Pour ce faire, plusieurs méthodes sont envisageables :

- Troncature du spectre de diffusion si celle-ci (la diffusion) ne chevauche pas avec le spectre de fluorescence
- Modélisation de la forme de la diffusion et retrait de la diffusion modélisée
- Utilisation d'outils chimiométriques comme l'ICA *(de l'anglais* Independent Component Analysis correspondant à une analyse en composantes indépendantes) qui permettent de différencier le signal de diffusion et le signal pur de fluorescence.

A partir de là, pour chaque mesure, on possède autant de spectres d'émission de fluorescence que de longueurs d'ondes (LEDs) d'excitation, et un spectre de transmittance infrarouge.

Il existe deux approches principales pour coupler les signaux. Elles diffèrent par l'ordre dans lequel on va d'une part coupler les informations et d'autre part réduire ces informations d'un nombre de variables de plusieurs milliers, à une ou deux dizaines, sans perte d'information utile. Différents modèles de calibration ou de classification pourront être appliqués à ces nouvelles variables en nombre réduit.

Les deux grandes approches sont donc les suivantes :

- Une approche bas niveau, qui couple les informations réduites, ou scores, issues de la décomposition de chaque spectre. Nous verrons plus loin les méthodes possibles de réduction de variables. On obtient alors un nombre restreint de variables qui contient quasiment la totalité de l'information initiale. Ces variables pourront alors être modélisées par différents algorithmes de calibration ou de classification.

- Une approche haut niveau, qui consiste à coupler les spectres eux-mêmes avant de procéder à l'étape de réduction de variables. La meilleure manière de coupler les informations spectrales est de concaténer les spectres. Puis diverses techniques de réduction de variables, comme ici encore l'analyse en composantes principales, pourront être appliquées. Les informations réduites combinant les deux technologies seront alors introduites dans les modèles de calibration, comme la régression multilinéaire, ou de classification.

Explorons maintenant plus en détail les diverses techniques qui pourraient être appliquées pour chacune de ces opérations.

La concaténation : les spectres sont rangés bout à bout, de préférence, chaque spectre d'émission de fluorescence selon les longueurs d'onde d'excitation croissantes, puis le spectre de transmittance infrarouge. Plusieurs précautions seront prises ici :

- Pour que chaque spectre ait le même poids au sein de l'ensemble, il convient de normaliser les spectres et de leur donner ainsi une intensité similaire. On peut par exemple opérer une normalisation par l'aire, une normalisation par l'écart-type du spectre suivi d'un centrage, ou encore une normalisation par le maximum afin que les intensités varient entre 0 et 1.

- Il faut veiller ensuite à ne pas générer de rupture entre le signal de la dernière longueur d'onde du spectre N-1 et le signal de la première longueur d'onde du spectre suivant N. Pour ce faire, diverses techniques sont possibles, comme de forcer la valeur à zéro si la partie concernée du spectre ne contient pas d'information. Il faudra malgré tout lisser le spectre pour obtenir un signal régulier.

Il s'agit de la méthode vue précédemment.

La réduction d'informations : cette réduction d'information à partir des variables spectrales s'appelle aussi décomposition du spectre. Chaque intensité à chaque longueur d'onde mesurée correspond à une variable, mais il existe une très grande redondance entre l'ensemble de ces variables (variables très corrélées). L'idée est donc d'extraire les informations indépendantes dont la somme recouvre l'ensemble de l'information initiale contenue dans le spectre.

La méthode la plus connue de décomposition est l'analyse en composantes principales qui assure que chaque nouvelle variable, appelée composante principale, constitue un vecteur orthogonal au système composé des autres composantes principales. Le nombre de composantes est déterminé par la capacité du modèle à expliquer la totalité de la variance spectrale. Toutefois, à partir d'un certain niveau de décomposition, cette variance ne contient plus que du bruit.

Dans le cas de structures multivoies comme la structure 3D de la fluorescence, on peut appliquer des techniques multivoies, telles que le PARAFAC (de l'anglais *PARAllel FACtor analysis*). Il s'agit d'identifier les structures 3D unitaires, ou facteurs, ou encore fluorophores unitaires, qui regroupés contiennent la totalité de la fluorescence acquise.

La calibration

Quelle que soit la technique de décomposition des spectres, unitaires ou concaténés, on obtient de nouvelles variables, appelées facteurs unitaires ou des composantes principales, en nombre limité, de l'ordre de 10 à 20 en général. Chaque échantillon est alors représenté par une combinaison linéaire de ces variables et par des poids spécifiques pour chacune d'elles. Ces poids portent généralement le nom de scores.

$$Echi = a_{i1} \times V_1 + a_{i2} \times V_2 + \dots a_{in} \times V_n + Constante$$

Où $a_{in}$ est le poids de chaque variable $V_n$ et n le nombre de variables obtenu lors de la décomposition.

Les scores caractérisent donc l'échantillon i pour cette décomposition.

Lors de la calibration, on utilise donc uniquement ces scores qu'on relie aux réponses d'intérêt que l'on souhaite prédire par la mesure spectrale.

Les méthodes de calibration les plus communes sont la PCR (de l'anglais *Principal Component Regression* correspondant à une régression sur composantes principales), ou encore la MLR (de l'anglais *Multiple Linear Regression* correspondant à une régression linéaire multiple) pour les calibrations de mode linéaire.

Il existe une autre méthode de régression linéaire, la PLS (de l'anglais *Partial Least Square* correspondant à une régression des moindres carrés partiels) qui présente comme particularité et avantage de réduire les spectres concaténés en nouvelles variables, tout en tenant compte des corrélations avec la réponse à calibrer et à prédire. Réduction et calibration se font donc en une seule étape.

On peut utiliser également des méthodes non linéaires comme des forêts aléatoires ou des techniques par proches voisins, ou encore des réseaux de neurones.

Exemple de réalisation du dispositif 1 d'analyse selon l'invention

Quel que soit l'exemple considéré dans la suite, le module 200 de traitement est un ordinateur mais il pourrait être n'importe quel dispositif équipé d'un processeur 220, tel que cela a été défini dans les sections précédentes. Quant au réseau de communication 300, il est filaire. Cela étant dit, il pourrait être de toute autre nature.

Dans la suite, on s'intéresse tout particulièrement aux éléments du module 100 de mesure qui ont été décrits dans la description détaillée.

Dans un exemple de réalisation du dispositif 1 d'analyse, l'élément optique 140 consiste en une paroi en verre distribuée par Edmund optics sous la référence 84479 et munie d'un diffuseur également distribué par Edmund optics sous la référence 83420. Le diffuseur consiste en un verre dépoli de haute qualité qui présente une rugosité suffisante pour créer de la diffusion. Le verre peut être dépoli en utilisant un procédé de sablage qui permet d'obtenir une diffusion uniforme sur la totalité de la surface.

Dans un exemple de réalisation du dispositif 1 d'analyser, la première source 122 d'excitation consiste en une lampe à incandescence halogène haute puissance et large bande distribuée par Newport sous la référence 6335. Elle émet un rayonnement électromagnétique dans l'infrarouge, entre 750 nm et 2500 nm.

Dans un exemple de réalisation du dispositif 1 d'analyse, la lentille de collimation 126 est distribuée par Newport sous la référence KBX139.

Dans un exemple de réalisation du dispositif 1 d'analyse, la deuxième source 132 d'excitation consiste en une pluralité de LEDs. Les LEDs sont au nombre de sept. des premières LEDs, quatre au total, émettent un rayonnement électromagnétique à une longueur d'onde de 275 nm et sont distribuées sous la référence CUD7GF1B par HTDS. Une deuxième LED émet un rayonnement électromagnétique à une longueur d'onde de 338 nm et est distribuée sous la référence CUD4AF1B par HTDS. Une troisième LED émet un rayonnement électromagnétique à une longueur d'onde de 285 nm et est vendue par HTDS sous la référence CUN8AF1B. Une quatrième LED émet un rayonnement électromagnétique à une longueur d'onde de 420 nm et est vendue par Roithner sous la référence LED420-01. L'ensemble des LEDs peuvent être solidarisées sur un support 136 fabriqué maison.

Dans un exemple de réalisation du dispositif 1 d'analyse, une lentille de collimation peut également être utilisée en combinaison avec la deuxième source 132. Une telle lentille de collimation est distribuée par Edmund optics sous la référence 49556.

Dans un exemple de réalisation du dispositif 1 d'analyse, le premier moyen 124 d'acquisition consiste en un spectromètre avaspec-2048XL distribué par Optoprim.

Dans un exemple de réalisation du dispositif 1 d'analyse, le deuxième moyen 134 d'acquisition consiste en un spectromètre avaspecULS-2048L distribué par Optoprim.

Dans un exemple de réalisation du dispositif 1 d'analyse, le réservoir 110 comprend une vitre détecteur d'échantillon référencée WW10530-B par Thorlabs. Le réservoir 110 peut également comprendre un détecteur de présence référencé VCNL4040M3OE par Mouser. Un tel équipement permet d'améliorer l'automatisation du procédé d'analyse selon l'invention.

Dans un exemple de réalisation du dispositif 1 d'analyse, le réservoir 110 peut être équipé d'un capteur de température. Le capteur de température peut consister en une vitre captant la température distribuée par Thorlabs sous la référence WW70530. Un autre capteur de température approprié est vendu par Mouser sous la référence MLX90614ESF-ACC-000-SP. Une connaissance de la température peut être très utile pour contrôler l'évolution de l'échantillon.

Exemple de mise en œuvre pratique du procédé de la présente invention

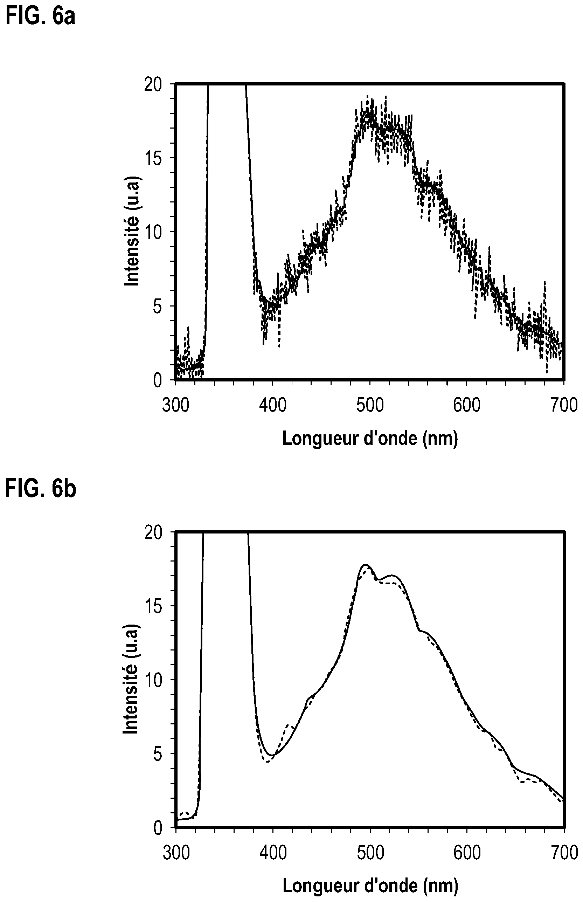

En référence à la figure 6a, le spectre brut obtenu lors de l'étape B) par application du procédé selon l'invention (en ligne solide) est comparé au spectre brut obtenu par spectroscopie de fluorescence au moyen de la méthode de référence connue de l'art antérieur (en ligne pointillée). L'échantillon dont il s'agit est de l'orge et se présente sous forme de grains. La longueur d'onde d'excitation utilisée pour réaliser ces mesures a été fixée à 340 nm. Comme cela peut être vu sur la figure 6a, le rapport signal sur bruit du spectre brut obtenu par la méthode de référence est nettement moins important que dans le spectre brut obtenu lors de l'étape B) par application du procédé selon l'invention.

La figure 6b montre le résultat de l'application d'un filtre numérique de type gaussien dont l'objectif est de filtrer le bruit électronique présent dans le signal sur le spectre brut obtenu lors de l'étape B) par application du procédé selon l'invention (en ligne solide) et sur le spectre brut obtenu par spectroscopie de fluorescence au moyen de la méthode de référence connue de l'art antérieur (en ligne pointillée).

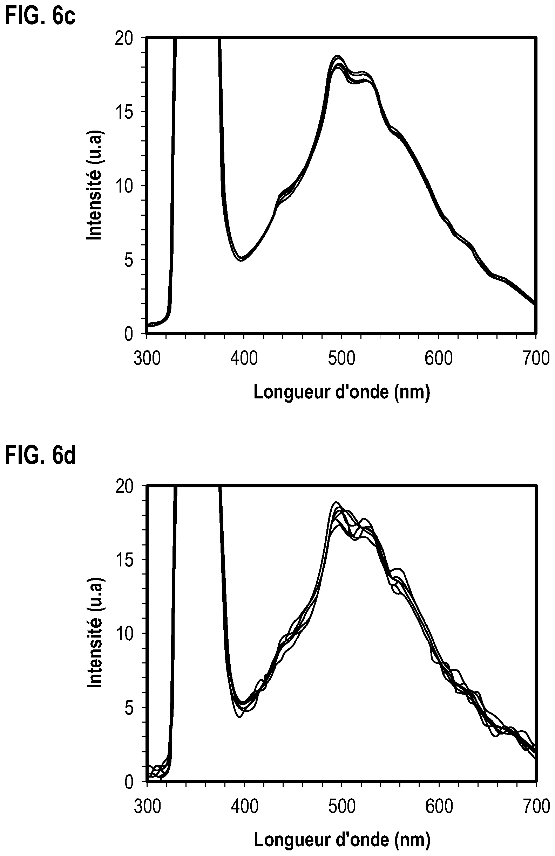

La figure 6c montre par ailleurs l'excellente répétabilité des mesures de fluorescence effectuées lors de la mise en œuvre du procédé selon l'invention sur le même échantillon. Elle est nettement meilleure que la répétabilité qui peut être obtenue en réalisant les mêmes mesures avec la méthode de référence (figure 6d) et ce malgré l'application préalable d'un filtre numérique. La très nette supériorité de la répétabilité des mesures s'explique par l'optimisation, au niveau de l'acquisition, du rapport signal sur bruit des spectres sur l'ensemble des composantes spectrales que permet la mise en œuvre du procédé selon l'invention.

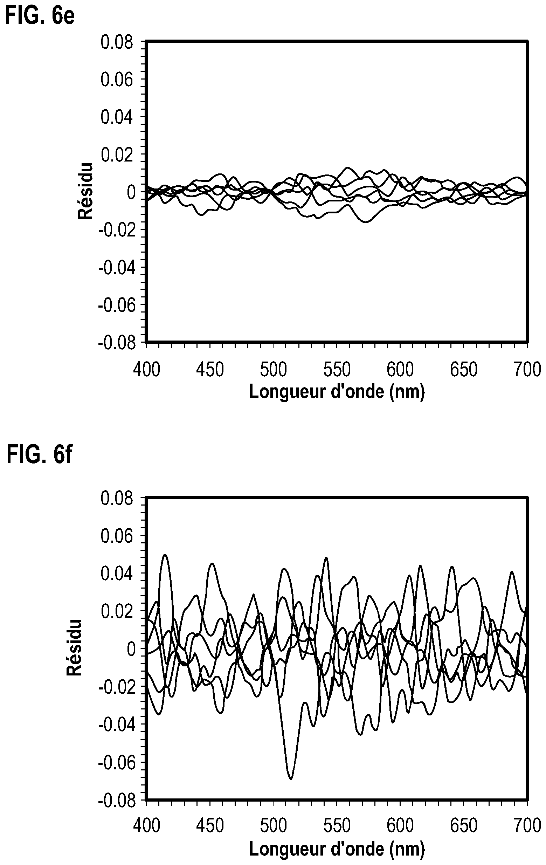

Aux figures 6e et 6f on peut voir que cela induit des différences importantes sur les résidus mesurés par rapport au spectre moyen selon le procédé mis en œuvre. En appliquant le procédé de l'invention d'une part il y a moins de variation des résidus et d'autre il y a moins d'étalement entre les résidus de chaque répétition.

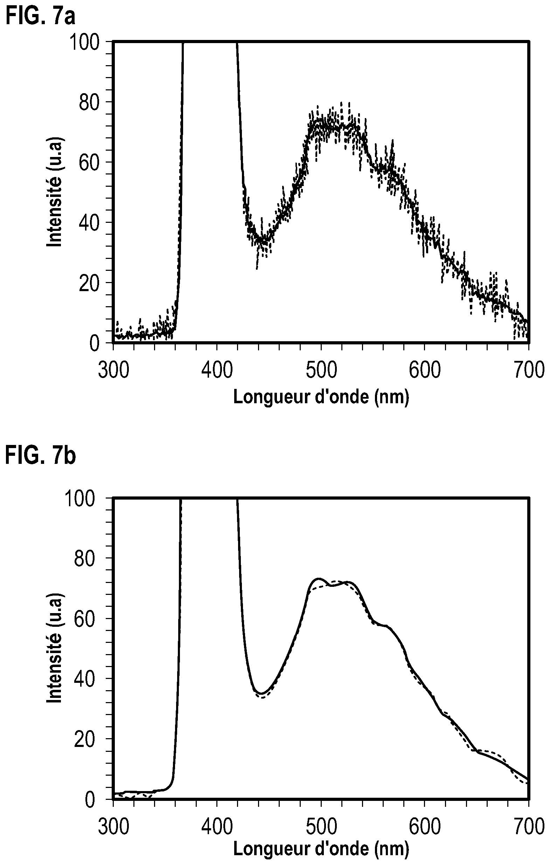

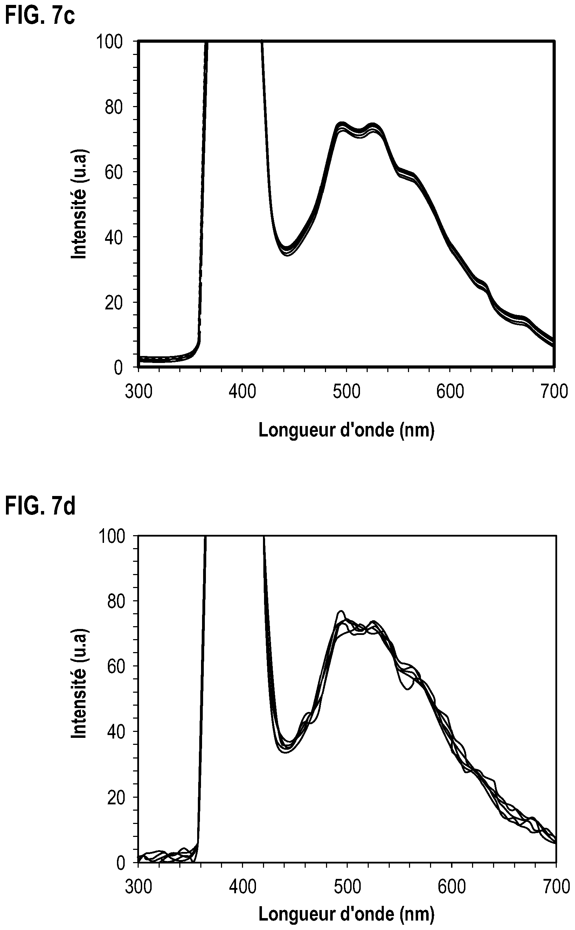

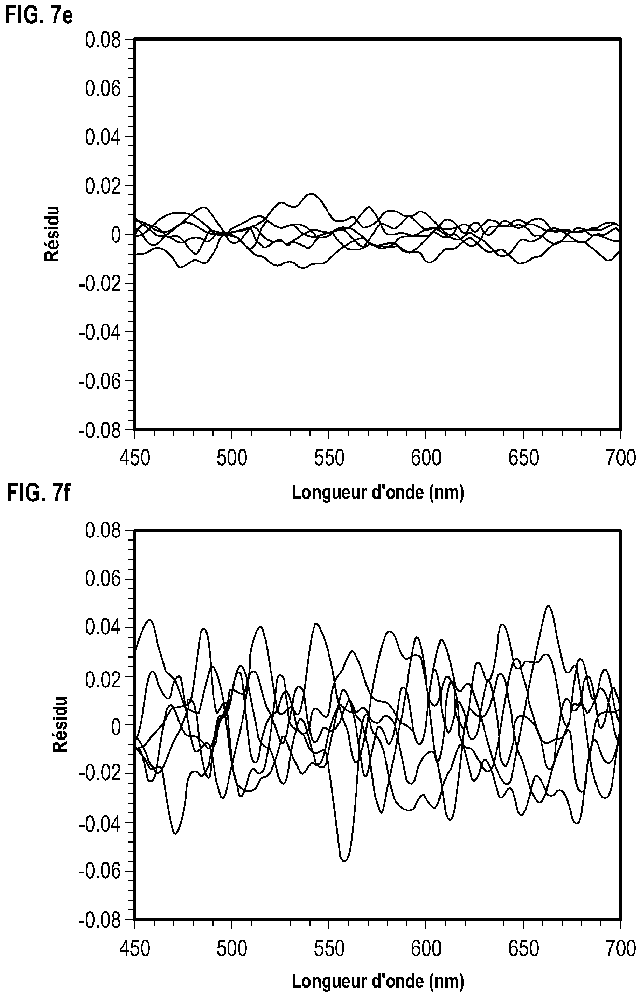

En référence aux figures 7a à 7f, on peut voir que les mêmes conclusions s'appliquent lorsque la longueur d'onde d'excitation est de 385 nm.

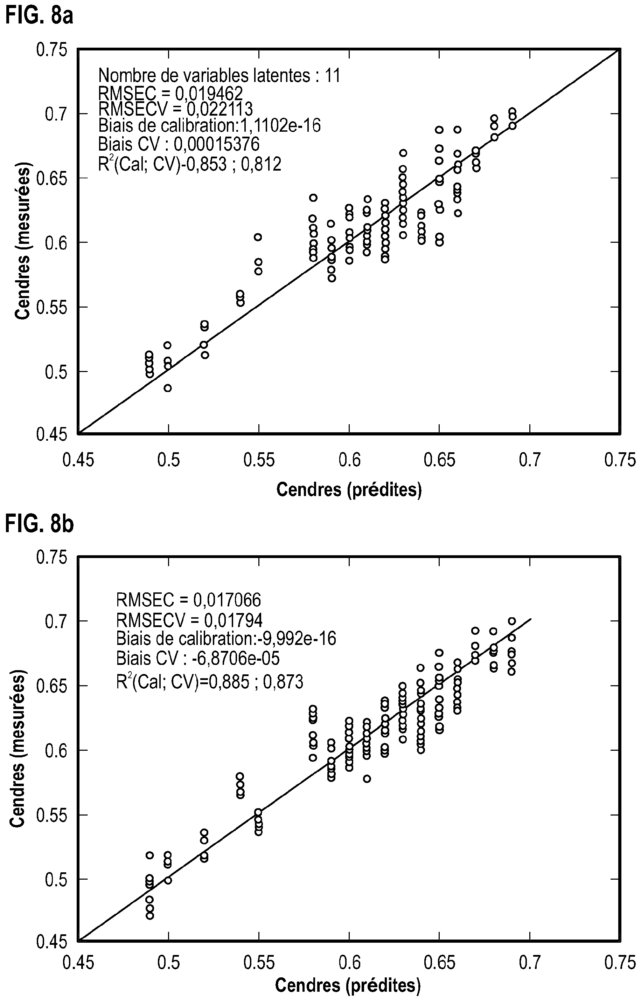

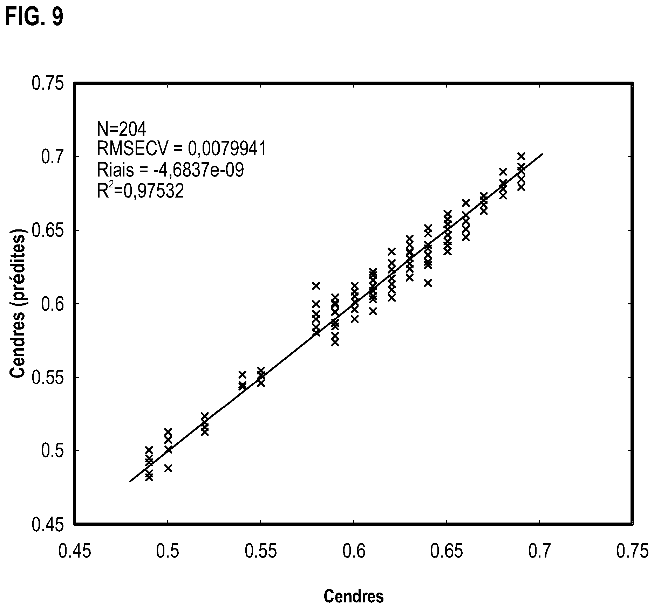

Le tableau ci-dessous présente de manière synthétique les résultats illustrés aux figures 8a, 8b et 9. Sur les figures 8a et 8b les 204 échantillons de farine ont été mesurées sur le même appareil, d'une part avec le module infrarouge et d'autre part avec le module fluorescence (séparément comme dans l'art antérieur). Les figures ci-dessous montrent les régressions de calibration obtenues en reliant en abscisse les valeurs mesurées par la méthode de référence pour chaque échantillon et en ordonnée les valeurs prédites en validation croisée à partir de la régression par les moindres carrés en fluorescence infrarouge ou de la régression linéaire multiple en spectroscopie de fluorescence, cette dernière étant elle-même construite à partir des scores de décomposition PARAFAC.

[Table 1]

| | Mesures par spectroscopie infrarouge (Fig. 8a) | Mesures par spectroscopie de Fluorescence (Fig. 8b) | **Mesures en chambre unique par spectroscopie IR et Fluo selon l'invention (Fig. 9)** |
|---|---|---|---|
| Calibration (RMSEC) | 0,020 | 0,017 | **0,005** |
| Validation croisée | 0,022 (RMSECV) | 0,018 (RMSECV) | **0,024 (RMSEP)** |
| Validation externe (RMSEP) | 0,065 | 0,085 | **0,023** |

L'abréviation:

- RMSEC désigne l'erreur d'étalonnage (*root mean square error of calibration* en anglais),
- RMSECV désigne l'écart-type de justesse (*root mean square error of cross-validation* en anglais), et
- RMSEP désigne l'erreur de prédiction (root mean square error of prediction en anglais).

Le tableau 1 montre une nette amélioration de l'erreur de calibration, avec une dégradation en prédiction externe dans le cas des deux technologies prises séparément, tandis que la dégradation est observée en validation croisée dans le cas du couplage. Ceci peut néanmoins s'expliquer par une légère sur-modélisation au cours de la calibration.

En tout état de cause, une amélioration de plus de 3 fois de la performance des prédictions externes est observée grâce au couplage des mesures par spectroscopie infrarouge et spectroscopie de fluorescence.

Les modes de réalisation montrés aux figures citées ne sont que des exemples possibles, nullement limitatifs, de l'invention qui englobe au contraire les variantes de formes et de conceptions à la portée de l'homme de l'art.

## Revendications

1. Dispositif (1) d'analyse d'un échantillon (S) hétérogène, ledit dispositif (1) comprenant

  - un module (100) de mesure comprenant :

    ∘ un réservoir (110, 410) configuré pour accueillir ledit échantillon (S) et muni d'une première paroi (112, 422) et d'une deuxième paroi (114, 432) opposée à la première paroi,
    ∘ un premier sous-ensemble (120) de spectroscopie infrarouge comprenant une première source (122) d'excitation configurée pour émettre un rayonnement électromagnétique ($E_{S1}$) dans le domaine de l'infrarouge et/ou du proche infrarouge vers la première paroi (112, 422) du réservoir, ladite première paroi (112, 422) étant transparente pour le rayonnement électromagnétique ($E_{S1}$) infrarouge, et un premier moyen (124) d'acquisition de spectres de transmittance ($S_{iR}$, $S_i$),
    ∘ un deuxième sous-ensemble (130) de spectroscopie de fluorescence comprenant au moins une deuxième source (132) d'excitation configurée pour émettre un rayonnement électromagnétique ($E_{S2}$) dans le domaine ultraviolet et/ou visible vers la deuxième paroi du réservoir, ladite deuxième paroi (114, 432) étant transparente pour les rayonnements électromagnétiques ($E_{S1}$, $E_{S2}$), de sorte que le volume de l'échantillon (S) susceptible d'être éclairé par la première source (122) d'excitation corresponde au moins en partie au volume de l'échantillon susceptible d'être éclairé par la deuxième source (132) d'excitation, et un deuxième moyen (134) d'acquisition de spectres de fluorescence ($S_{f1}$, $S_{f2}$) dudit échantillon (S), le premier sous-ensemble (120) comprenant un élément optique (140) diffusant et transparent pour le rayonnement électromagnétique ($E_{S1}$) émis par ladite première source (122) d'excitation, ledit élément optique (140) étant positionné entre la première source (122) d'excitation et la première paroi (112, 422) du réservoir ou entre la deuxième paroi du réservoir (114, 432) et le premier moyen (124) d'acquisition, hors d'un trajet optique du rayonnement électromagnétique ($E_{S2}$) émis par la deuxième source (132) d'excitation et hors d'un angle solide de collecte d'un signal de fluorescence émis par l'échantillon (S) lorsqu'il est exposé au rayonnement électromagnétique ($E_{S2}$) émis par la deuxième source (132) d'excitation, et

  - un module (200) de traitement connecté au module (100) de mesure par un réseau de communication (300) et comprenant un processeur (220) configuré pour analyser les données obtenues par spectroscopie infrarouge et spectroscopie de fluorescence,
  - un système de retenue (400) et une ouverture (150) du module (100) de mesure servant à y distribuer l'échantillon (S) ou un logement recevant le système de retenue (400) de l'échantillon, ledit logement comprenant des faces intérieures sur lesquelles est plaqué le système de retenue (400), le système de retenue (400) de l'échantillon comportant :

◦ le réservoir (410), ledit réservoir (410) comprenant une première partie vitrée (420) munie de la première paroi (422) et une deuxième partie vitrée (430) munie de la deuxième paroi (432), ladite première partie vitrée (420) étant montée de manière amovible sur ladite deuxième partie vitrée (430),
◦ un bloc support (450) configuré pour positionner le système de retenue (400) dans ledit logement du module (100) de mesure ou au niveau de l'ouverture (150) servant à y distribuer l'échantillon (S), le bloc support (450) comprenant une portion de raccordement (460) et une base (454) de préhension du système de retenue (400) formant un coude avec la portion de raccordement (460) dans le dispositif (1),
◦ une portion amovible (470) comprenant un logement (472) recevant le réservoir (410), ladite deuxième partie vitrée (430) étant fixée à la portion amovible (470), ladite portion amovible (470) étant reliée de manière amovible par rapport à la portion de raccordement (460), la base (454) de préhension venant en butée dans le logement du module (100) de mesure ou se positionnant au niveau de l'ouverture (150) du module (100) de mesure, et permettant à la portion de raccordement (460) et à la portion amovible (470) de s'étendre dans le module (100), sur le chemin optique, lorsque le système de retenue (400) est inséré dans le module (100) de mesure,
◦ une portion articulée (480) comprenant une ouverture (482), ladite portion articulée (480) étant montée pivotante sur le bloc support (450) et apte à passer d'une position de montage dans laquelle elle est éloignée de la portion amovible (470) à une position d'utilisation dans laquelle elle est rabattue sur la portion amovible (470), ladite ouverture (482) étant en vis-à-vis du réservoir (410), ladite portion articulée (480) comprenant des moyens (486a, 486b, 488a, 488b) compressibles permettant le plaquage du système de retenue (400) contre les faces intérieures dudit logement du module (100) de mesure recevant le système de retenue (400) lorsque ladite portion articulée (480) est en position d'utilisation.

2. Dispositif (1) selon la revendication 1, dans lequel l'élément optique (140) constitue la première paroi (112, 422) dudit réservoir.

3. Dispositif (1) selon l'une des revendications 1 ou 2, dans lequel la première paroi (112, 422) du réservoir est mobile le long d'un axe (X) orthogonal à un plan passant par la première paroi (112, 422).

4. Dispositif (1) selon l'une quelconque des revendications 1 à 3, dans lequel la deuxième paroi (114, 432) du réservoir (110, 410) est anti-réfléchissante pour le rayonnement électromagnétique ($E_{S2}$) émis par la deuxième source (132).

5. Dispositif (1) selon l'une quelconque des revendications précédentes, dans lequel ladite deuxième source (132) est située entre la deuxième paroi (114, 432) et le premier moyen (124) d'acquisition.

6. Dispositif (1) selon l'une quelconque des revendications précédentes, dans lequel un axe (X') passant par le deuxième moyen (134) d'acquisition et un plan médian et sensiblement orthogonal à la deuxième paroi (114) du réservoir forme un angle $\alpha$ par rapport à un axe (X) passant par la première source (122) d'excitation et le deuxième moyen d'acquisition (134).

7. Dispositif (1) selon l'une quelconque des revendications précédentes, dans lequel la première source (122) d'excitation émet un rayonnement électromagnétique ($E_{S1}$) polychromatique large-bande, ladite première source (122) consistant en une source à incandescence halogène haute puissance.

8. Dispositif (1) selon l'une quelconque des revendications précédentes, dans lequel la(es) deuxième(s) source(s) (132) d'excitation émet(tent) un rayonnement électromagnétique ($E_{S2}$) monochromatique, ladite(lesdites) deuxième(s) source(s) (132) consistant en une(des) diode(s) électroluminescente(s).

9. Procédé d'analyse d'un échantillon (S) hétérogène avec un dispositif (1) selon l'une quelconque des revendications précédentes, ledit procédé comprenant les étapes suivantes :

A) acquérir un spectre de transmittance ($S_i$) dudit échantillon (S) avec le premier sous-ensemble (120) de spectroscopie infrarouge,
B) acquérir des spectres de fluorescence ($S_{f1}$, $S_{f2}$) dudit échantillon (S) avec le deuxième sous-ensemble (130) de spectroscopie de fluorescence,

C) analyser les données obtenues par spectroscopie infrarouge et spectroscopie de fluorescence au moyen du module de traitement (200), un processeur (220) étant configuré pour déterminer au moins un critère caractérisant ledit échantillon à partir des données issues de l'analyse,

D) coupler les données obtenues par spectroscopie infrarouge et spectroscopie de fluorescence, au niveau spectral, au moyen du module de traitement, par concaténation des spectres, traités préalablement, et par association de scores issus de la décomposition de chaque spectre, pour construire des régressions linéaires ou des modèles non linéaires et obtenir des calibrations d'un critère descriptif de l'état de l'échantillon, comme un critère de qualité technologique, sensoriel ou nutritionnel et sanitaire.

**Patentansprüche**

1. Vorrichtung (1) zur Analyse einer heterogenen Probe (S), wobei die Vorrichtung (1) umfasst

- ein Messmodul (100), welches umfasst:

- einen Behälter (110, 410), der so konfiguriert ist, dass er die Probe (S) aufnimmt, und mit einer ersten Wand (112, 422) und einer der ersten Wand gegenüberliegenden zweiten Wand (114, 432) versehen ist,
- eine erste Unterbaugruppe (120) für Infrarotspektroskopie, die eine erste Anregungsquelle (122) umfasst, die so konfiguriert ist, dass sie eine elektromagnetische Strahlung ($E_{S1}$) im Infrarot- und/oder Nahinfrarotbereich in Richtung der ersten Wand (112, 422) des Behälters emittiert, wobei die erste Wand (112, 422) für die elektromagnetische Infrarotstrahlung ($E_{S1}$) durchlässig ist, und ein erstes Mittel (124) zum Erfassen von Transmissionsspektren ($S_{iR}$, $S_i$),
- eine zweite Unterbaugruppe (130) für Fluoreszenzspektroskopie, die mindestens eine zweite Anregungsquelle (132) umfasst, die so konfiguriert ist, dass sie eine elektromagnetische Strahlung ($E_{S2}$) im ultravioletten und/oder sichtbaren Bereich in Richtung der zweiten Wand des Behälters emittiert, wobei die zweite Wand (114, 432) für die elektromagnetischen Strahlungen ($E_{S1}$, $E_{S2}$) durchlässig ist, sodass das Volumen der Probe (S), das von der ersten Anregungsquelle (122) beleuchtet werden kann, mindestens zum Teil dem Volumen der Probe entspricht, das von der zweiten Anregungsquelle (132) beleuchtet werden kann, und ein zweites Mittel (134) zum Erfassen von Fluoreszenzspektren (Sf1, Sf2) der Probe (S), wobei die erste Unterbaugruppe (120) ein optisches Element (140) umfasst, das die von der ersten Anregungsquelle (122) emittierte elektromagnetische Strahlung ($E_{S1}$) streut und durchlässt, wobei das optische Element (140) zwischen der ersten Anregungsquelle (122) und der ersten Wand (112, 422) des Behälters oder zwischen der zweiten Wand des Behälters (114, 432) und dem ersten Erfassungsmittel (124) positioniert ist, außerhalb eines optischen Weges der von der zweiten Anregungsquelle (132) emittierten elektromagnetischen Strahlung ($E_{S2}$) und außerhalb eines Raumwinkels zum Sammeln eines Fluoreszenzsignals, das von der Probe (S) emittiert wird, wenn sie der von der zweiten Anregungsquelle (132) emittierten elektromagnetischen Strahlung ($E_{S2}$) ausgesetzt ist, und

- ein Verarbeitungsmodul (200), das über ein Kommunikationsnetzwerk (300) mit dem Messmodul (100) verbunden ist und einen Prozessor (220) umfasst, der so konfiguriert ist, dass er die durch Infrarotspektroskopie und Fluoreszenzspektroskopie erhaltenen Daten analysiert,
- ein Rückhaltesystem (400) und eine Öffnung (150) des Messmoduls (100), die dazu dient, die Probe (S) darin zu verteilen, oder eine Aufnahme, die das Rückhaltesystem (400) für die Probe aufnimmt, wobei die Aufnahme Innenseiten umfasst, an die das Rückhaltesystem (400) gedrückt wird, wobei das Rückhaltesystem (400) für die Probe umfasst:

- den Behälter (410), wobei der Behälter (410) einen ersten verglasten Teil (420), der mit der ersten Wand (422) versehen ist, und einen zweiten verglasten Teil (430), der mit der zweiten Wand (432) versehen ist, umfasst, wobei der erste verglaste Teil (420) abnehmbar an dem zweiten verglasten Teil (430) montiert ist,
- einen Trägerblock (450), der so konfiguriert ist, dass er das Rückhaltesystem (400) in der Aufnahme des Messmoduls (100) oder auf Höhe der Öffnung (150) positioniert, die dazu dient, die Probe (S) darin zu verteilen, wobei der Trägerblock (450) einen Verbindungsabschnitt (460) und eine Basis (454) zum Greifen des Rückhaltesystems (400) umfasst, die mit dem Verbindungsabschnitt (460) in der Vorrichtung (1) einen Bogen bildet,
- einen abnehmbaren Abschnitt (470), der eine Aufnahme (472) umfasst, welche den Behälter (410) aufnimmt, wobei der zweite verglaste Teil (430) an dem abnehmbaren Abschnitt (470) befestigt ist, wobei der abnehmbare Abschnitt (470) abnehmbar in Bezug auf den Verbindungsabschnitt (460) verbunden ist, wobei die Basis (454) zum Greifen in der Aufnahme des Messmoduls (100) in Anlage kommt oder sich auf Höhe der Öffnung (150) des Messmoduls (100) positioniert, und es dem Verbindungsabschnitt (460) und dem abnehmbaren Abschnitt (470) ermöglicht, sich in dem Modul (100) auf dem optischen Weg zu erstrecken, wenn das Rückhaltesystem (400) in das Messmodul (100) eingesetzt ist,
- einen angelenkten Abschnitt (480), der eine Öffnung (482) umfasst, wobei der angelenkte Abschnitt (480) schwenkbar an dem Trägerblock (450) montiert und in der Lage ist, von einer Montageposition, in der er von dem abnehmbaren Abschnitt (470) entfernt ist, in eine Gebrauchsposition, in der er auf den abnehmbaren Abschnitt (470) geklappt ist, überzugehen, wobei die Öffnung (482) dem Behälter (410) gegenüberliegt, wobei der angelenkte Abschnitt (480) komprimierbare Mittel (486a, 486b, 488a, 488b) umfasst, die das Andrücken des Rückhaltesystems (400) an die Innenseiten der Aufnahme des Messmoduls (100), die das Rückhaltesystem (400) aufnimmt, ermöglichen, wenn sich der angelenkte Abschnitt (480) in Gebrauchsposition befindet.

2. Vorrichtung (1) nach Anspruch 1, wobei das optische Element (140) die erste Wand (112, 422) des Behälters bildet.

3. Vorrichtung (1) nach einem der Ansprüche 1 oder 2, wobei die erste Wand (112, 422) des Behälters entlang einer Achse (X), die zu einer durch die erste Wand (112, 422) verlaufenden Ebene orthogonal ist, beweglich ist.

4. Vorrichtung (1) nach einem der Ansprüche 1 bis 3, wobei die zweite Wand (114, 432) des Behälters (110, 410) für die von der zweiten Quelle (132) emittierte elektromagnetische Strahlung ($E_{S2}$) antireflektierend ist.

5. Vorrichtung (1) nach einem der vorstehenden Ansprüche, wobei sich die zweite Quelle (132) zwischen der zweiten Wand (114, 432) und dem ersten Erfassungsmittel (124) befindet.

6. Vorrichtung (1) nach einem der vorstehenden Ansprüche, wobei eine Achse (X'), die durch das zweite Erfassungsmittel (134) und eine Mittelebene und im Wesentlichen orthogonal zu der zweiten Wand (114) des Behälters verläuft, einen Winkel $\alpha$ in Bezug auf eine Achse (X) bildet, die durch die erste Anregungsquelle (122) und das zweite Erfassungsmittel (134) verläuft.

7. Vorrichtung (1) nach einem der vorstehenden Ansprüche, wobei die erste Anregungsquelle (122) eine breitbandige polychromatische elektromagnetische Strahlung ($E_{S1}$) emittiert, wobei die erste Quelle (122) aus einer Hochleistungs-Halogenglühbirnen-Quelle besteht.

8. Vorrichtung (1) nach einem der vorstehenden Ansprüche, wobei die zweite(n) Anregungsquelle(n) (132) eine monochromatische elektromagnetische Strahlung ($E_{S2}$) emittiert/emittieren, wobei die zweite(n) Quelle(n) (132) aus einer oder mehreren Leuchtdioden besteht/bestehen.

9. Verfahren zur Analyse einer heterogenen Probe (S) mit einer Vorrichtung (1) nach einem der vorstehenden Ansprüche, wobei das Verfahren die folgenden Schritte umfasst:

A) Erfassen eines Transmissionsspektrums ($S_i$) der Probe (S) mit der ersten Unterbaugruppe (120) für Infrarotspektroskopie,
B) Erfassen von Fluoreszenzspektren ($S_{f1}$, $S_{f2}$) der Probe (S) mit der zweiten Unterbaugruppe (130) für Fluoreszenzspektroskopie,
C) Analysieren der durch Infrarotspektroskopie und Fluoreszenzspektroskopie erhaltenen Daten mittels des Verarbeitungsmoduls (200), wobei ein Prozessor (220) so konfiguriert ist, dass er mindestens ein Kriterium, das die Probe charakterisiert, auf Grundlage der aus der Analyse stammenden Daten bestimmt,
D) Koppeln der durch Infrarotspektroskopie und Fluoreszenzspektroskopie erhaltenen Daten auf spektraler Ebene mittels des Verarbeitungsmoduls durch Verkettung der zuvor verarbeiteten Spektren und durch Zuordnung von Punktewerten, die aus der Zerlegung jedes Spektrums stammen, um lineare Regressionen oder nichtlineare Modelle zu konstruieren und Kalibrierungen eines Kriteriums zu erhalten, das den Zustand der Probe beschreibt, wie etwa ein technologisches, sensorisches oder nährstoffliches und gesundheitliches Qualitätskriterium.

## Claims

1. A device (1) for analysing a heterogeneous sample (S), said device (1) comprising

- a measurement module (100) comprising:

o a reservoir (110, 410) configured to accommodate said sample (S) and equipped with a first wall (112, 422) and a second wall (114, 432) opposite the first wall,

o a first infrared spectroscopy subassembly (120) comprising a first excitation source (122) configured to emit electromagnetic radiation ($E_{S_1}$) in the infrared and/or near-infrared field to the first wall (112, 422) of the reservoir, said first wall (112, 422) being transparent for the infrared electromagnetic radiation ($E_{S_1}$), and a first means (124) for acquiring transmittance spectra ($S_{iR}$, $S_i$),

o a second fluorescence spectroscopy sub-assembly (130) comprising at least a second excitation source (132) configured to emit electromagnetic radiation ($E_{S_2}$) in the ultraviolet and/or visible field to the second wall of the reservoir, said second wall (114, 432) being transparent for the electromagnetic radiation ($E_{S_1}$, $E_{S_2}$), such that the volume of the sample (S) which can be illuminated by the first excitation source (122) corresponds at least partially to the volume of the sample which can be illuminated by the second excitation source (132), and a second means (134) for acquiring fluorescence spectra ($S_{f1}$, $S_{f2}$) of said sample (S),

the first subassembly (120) comprising a diffusing and transparent optical element (140) for the electromagnetic radiation ($E_{S_1}$) emitted by said first excitation source (122), said optical element (140) being positioned between the first excitation source (122) and the first wall (112, 422) of the reservoir or between the second wall of the reservoir (114, 432) and the first acquisition means (124), outside an optical path of the electromagnetic radiation ($E_{S_2}$) emitted by the second excitation source (132) and outside a solid angle for collecting a fluorescence signal emitted by the sample (S) when the electromagnetic radiation ($E_{S_2}$) emitted by the second excitation source (132) is exposed, and

- a processing module (200) connected to the measurement module (100) by a communication network (300) and comprising a processor (220) configured to analyse the data obtained by infrared spectroscopy and fluorescence spectroscopy,

- a retaining system (400) and an opening (150) in the measurement module (100) used to dispense the sample (S) into it, or a housing receiving the retaining system (400) of the sample, said housing comprising inner surfaces against which the retaining system (400) is pressed, the retaining system (400) of the sample comprising:

○ the reservoir (410), said reservoir (410) comprising a first glazed part (420) equipped with the first wall (422) and a second glazed part (430) equipped with the second wall (432), said first glazed part (420) being removably mounted on said second glazed part (430),

○ a support block (450) configured to position the retaining system (400) within said housing of the measuring module (100) or at the opening (150) used to dispense the sample (S) into it, the support block (450) comprising a connection portion (460) and a base (454) for gripping the retaining system (400), which forms a bend with the connection portion (460) in the device (1),

○ a removable portion (470) comprising a housing (472) receiving the reservoir (410), said second glazed part (430) being fixed to the removable portion (470), said removable portion (470) being removably connected with respect to the connection portion (460), the gripping base (454) abutting against the housing of the measuring module (100) or positioning itself at the opening (150) of the measuring module (100), and allowing the connection portion (460) and the removable portion (470) to extend into the module (100), in the optical path, when the retaining system (400) is inserted into the measurement module (100),

○ an articulated portion (480) comprising an opening (482), said articulated portion (480) being pivotally mounted on the support block (450) and capable of moving from a mounting position wherein it is away from the removable portion (470) to a use position wherein it is folded down on the removable portion (470), said opening (482) being opposite the reservoir (410), said articulated portion (480) comprising compressible means (486a, 486b, 488a, 488b) allowing the flattening the retaining system (400) against the inner faces of said housing of the measuring module (100) receiving the retaining system (400) when said articulated portion (480) is in the position of use.

2. The device (1) according to claim 1, wherein the optical element (140) constitutes the first wall (112, 422) of said reservoir.

3. The device (1) according to one of claims 1 or 2, wherein the first wall (112, 422) of the reservoir is movable along an axis (X) orthogonal to a plane passing through the first wall (112, 422).

4. The device (1) according to any one of claims 1 to 3, wherein the second wall (114, 432) of the reservoir (110, 410) is anti-reflective for the electromagnetic radiation ($E_{S_2}$) emitted by the second source (132).

5. The device (1) according to any one of the preceding claims, wherein said second source (132) is located between the second wall (114, 432) and the first acquisition means (124).

6. The device (1) according to any one of the preceding claims, wherein an axis (X') passing through the second acquisition means (134) and a median plane substantially orthogonal to the second wall (114) of the reservoir forms an angle $\alpha$ with respect to an axis (X) passing through the first excitation source (122) and the second acquisition means (134).

7. The device (1) according to any of the preceding claims, wherein the first excitation source (122) emits broadband polychromatic electromagnetic radiation ($E_{S1}$), said first source (122) consisting of a high-power halogen incandescent source.

8. The device (1) according to any of the preceding claims, wherein the second excitation source(s) (132) emit(s) monochromatic electromagnetic radiation ($E_{S2}$), said second source(s) (132) consisting of one or more light-emitting diode(s).

9. A method for analysing a heterogeneous sample (S) with a device (1) according to any one of the preceding claims, said method comprising the following steps:

A) acquiring a transmittance spectrum ($S_i$) of said sample (S) with the first infrared spectroscopy subassembly (120),
B) acquiring fluorescence spectra ($S_{f1}$, $S_{f2}$) of said sample (S) using the second fluorescence spectroscopy subsystem (130),
C) analysing the data obtained by infrared spectroscopy and fluorescence spectroscopy using the processing module (200), a processor (220) being configured to determine at least one criterion characterising said sample from the data resulting from the analysis,
D) coupling the data obtained by infrared spectroscopy and fluorescence spectroscopy, at the spectral level, by means of the processing module, by concatenation of spectra, processed beforehand, and by association of scores coming from the breakdown of each spectrum, for construct linear regressions or non-linear models and obtain calibrations of a descriptive criterion of the state of the sample, like a technological, sensorial or nutritional and sanitary quality criterion.

## FIG. 1a

## FIG. 1b

## FIG. 1c

**FIG. 2a**

**FIG. 2b**

## FIG. 3

## FIG. 4a

**FIG. 4b**

454

400

460

470

480

432

410

**FIG. 5**

410

422

420

430

470

432

## FIG. 6a

Longueur d'onde (nm)

## FIG. 6b

Longueur d'onde (nm)

**FIG. 6c**

**FIG. 6d**

**FIG. 6e**

**FIG. 6f**

**FIG. 7a**

**FIG. 7b**

**FIG. 7c**

**FIG. 7d**

**FIG. 7e**

**FIG. 7f**

## FIG. 8a

Nombre de variables latentes : 11
RMSEC = 0,019462
RMSECV = 0,022113
Biais de calibration:1,1102e-16
Biais CV : 0,00015376
$R^2$(Cal; CV)-0,853 ; 0,812

Cendres (mesurées)

Cendres (prédites)

## FIG. 8b

RMSEC = 0,017066
RMSECV = 0,01794
Biais de calibration:-9,992e-16
Biais CV : -6,8706e-05
$R^2$(Cal; CV)=0,885 ; 0,873

Cendres (mesurées)

Cendres (prédites)

FIG. 9

N=204
RMSECV = 0,0079941
Riais = -4,6837e-09
$R^2$=0,97532

Cendres (prédites)

Cendres

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2019118800 A1
- EP 1850117 A1
- FR 3047313 A1
- WO 2020012029 A1